# EUROPÄISCHE PATENTSCHRIFT

(11) **EP 1 516 188 B1**
(45) Veröffentlichungstag und Bekanntmachung des Hinweises auf die Patenterteilung: **15.11.2006**
(21) Anmeldenummer: 03760571.4
(22) Anmeldetag: 24.06.2003
(51) Int. Cl.: G01N 33/569, G01N 33/92

(54) **VERFAHREN ZUM NACHWEIS UND ZUR ENTFERNUNG VON ENDOTOXIN**
METHOD FOR IDENTIFYING AND EXTRACTING ENDOTOXIN
PROCEDE DE DETECTION ET D'EXTRACTION D'ENDOTOXINES

(30) Priorität: 24.06.2002 DE 10228133; 24.02.2003 DE 10307793
(43) Veröffentlichungstag der Anmeldung: 23.03.2005
(73) Patentinhaber: Profos AG, 93053 Regensburg (DE)
(72) Erfinder: SCHÜTZ, Michael, 93138 Kareth-Lappersdorf (DE); MEYER, Roman, 92287 Schmidmühlen (DE); GRALLERT, Holger, 93080 Pentling (DE); MILLER, Stefan, 93053 Regensburg (DE)
(74) Vertreter: Dehmel, Albrecht
(86) Internationale Anmeldenummer: PCT/DE2003/002096
(87) Internationale Veröffentlichungsnummer: WO 2004/001418

(56) Entgegenhaltungen:
- WO-A-03/000888
- NESPER JUTTA ET AL: "Characterization of Vibrio cholerae O1 antigen as the bacteriophage K139 receptor and identification of IS1004 insertions aborting O1 antigen biosynthesis" JOURNAL OF BACTERIOLOGY, Bd. 182, Nr. 18, September 2000 (2000-09), Seiten 5097-5104, XP002277663 ISSN: 0021-9193
- RUDOLPH ALAN S ET AL: "Comparative study of the accurate measurement of endotoxin in liposome encapsulated hemoglobin" PROCEEDINGS OF THE 11TH CONGRESS OF THE INTERNATIONAL SOCIETY FOR ARTIFICIAL CELLS, BLOOD SUBSTITUTES AND IMMOBILIZATION BIOTECHNOLOGY, (ISABI);BOSTON, MA, USA JUL 24-27 1994, Bd. 22, Nr. 5, 24. Juli 1994 (1994-07-24), Seite A153 XP008030016 Artif Cells Blood Substitutes Immobilization Biotechnol;Artificial Cells, Blood Substitutes, and Immobilization Biotechnology Nov 1994 Marcel Dekker Inc, New York, NY, USA
- SUN W ET AL: "USE OF BIOLUMINESCENT SALMONELLA FOR ASSESSING THE EFFICIENCY OF CONSTRUCTED PHAGE-BASED BIOSORBENT" JOURNAL OF INDUSTRIAL MICROBIOLOGY AND BIOTECHNOLOGY, BASINGSTOKE, GB, Bd. 25, Nr. 5, November 2000 (2000-11), Seiten 273-275, XP008016601 ISSN: 1367-5435

### SEQUENCE LISTING

<110> PROFOS AG
<120> Verfahren zum Nachweis und zur Entfernung von Endotoxin
<130> PRO-008 PCT
<140> unknown
<141> 2003-06-24
<150> 102 28 133.5
<151> 2002-06-24
<150> 103 07 793.6
<151> 2003-02-24
<160> 8
<170> PatentIn version 3.1
<210> 1
<211> 78
<212> DNA
<213> künstlich hergestellte Sequenz
<400> 1
   gaaggaacta gtcatatggc tagctggagc cacccgcagt tcgaaaaagg cgccagtaat     60
   aatacatatc aacacgtt                                                   78
<210> 2
<211> 54
<212> DNA
<213> künstlich hergestellte Sequenz
<400> 2
   acgcgcaaag cttgtcgacg gatcctatca ttcttttacc ttaattatgt agtt 54
<210> 3
<211> 78
<212> DNA
<213> künstlich hergestellte Sequenz
<400> 3
   gaaggaacta gtcatatggc ttgttggagc cacccgcagt tcgaaaaagg cgccagtaat      60
   aatacatatc aacacgtt                                                    78
<210> 4
<211> 78
<212> DNA
<213> künstlich hergestellte Sequenz
<400> 4
   gaaggaacta gtcatatggc tagctggagc cacccgcagt tcgaaaaagg cgcctgtaat      60
   aatacatatc aacacgtt                                                    78
<210> 5
<211> 19
<212> PRT
<213> künstlich hergestellte Sequenz
<400> 5
   Met Ala Ser Trp Ser His Pro Gln Phe Glu Lys Gly Ala Ser Asn Asn
   1               5                   10                  15
   Thr Tyr Gln
<210> 6
<211> 19
<212> PRT
<213> künstlich hergestellte Sequenz
<400> 6
   Met Ala Cys Trp Ser His Pro Gln Phe Glu Lys Gly Ala Ser Asn Asn
   1               5                   10                  15
   Thr Tyr Gln
<210> 7
<211> 19
<212> PRT
<213> künstlich hergestellte Sequenz
<400> 7
   Met Ala Ser Trp Ser His Pro Gln Phe Glu Lys Gly Ala Cys Asn Asn
   1               5                   10                  15
   Thr Tyr Gln
<210> 8
<211> 539
<212> PRT
<213> künstlich hergestellte Sequenz
<400> 8
   Met Ala Ser Trp Ser His Pro Gln Phe Glu Lys Gly Ala Ser Asn Asn
   1               5                   10                  15
   Thr Tyr Gln His Val Ser Asn Glu Ser Arg Tyr Val Lys Phe Asp Pro
               20                  25                  30
   Thr Asp Thr Asn Phe Pro Pro Glu Ile Thr Asp Val Gln Ala Ala Ile
           35                  40                  45
   Ala Ala Ile Ser Pro Ala Gly Val Asn Gly Val Pro Asp Ala Ser Ser
       50                  55                  60
   Thr Thr Lys Gly Ile Leu Phe Leu Ala Thr Glu Gln Glu Val Ile Asp
   65                  70                  75                  80
   Gly Thr Asn Asn Thr Lys Ala Val Thr Pro Ala Thr Leu Ala Thr Arg
                   85                  90                  95
   Leu Ser Tyr Pro Asn Ala Thr Glu Ala Val Tyr Gly Leu Thr Arg Tyr
               100                 105                 110
   Ser Thr Asp Asp Glu Ala Ile Ala Gly Val Asn Asn Glu Ser Ser Ile
           115                 120                 125
   Thr Pro Ala Lys Phe Thr Val Ala Leu Asn Asn Val Phe Glu Thr Arg
       130                 135                 140
   Val Ser Thr Glu Ser Ser Asn Gly Val Ile Lys Ile Ser Ser Leu Pro
   145                 150                 155                 160
   Gln Ala Leu Ala Gly Ala Asp Asp Thr Thr Ala Met Thr Pro Leu Lys
                   165                 170                 175
   Thr Gln Gln Leu Ala Val Lys Leu Ile Ala Gln Ile Ala Pro Ser Lys
               180                 185                 190
   Asn Ala Ala Thr Glu Ser Glu Gln Gly Val Ile Gln Leu Ala Thr Val
           195                 200                 205
   Ala Gln Ala Arg Gln Gly Thr Leu Arg Glu Gly Tyr Ala Ile Ser Pro
       210                 215                 220
   Tyr Thr Phe Met Asn Ser Thr Ala Thr Glu Glu Tyr Lys Gly Val Ile
   225                 230                 235                 240
   Lys Leu Gly Thr Gln Ser Glu Val Asn Ser Asn Asn Ala Ser Val Ala
                   245                 250                 255
   Val Thr Gly Ala Thr Leu Asn Gly Arg Gly Ser Thr Thr Ser Met Arg
               260                 265                 270
   Gly Val Val Lys Leu Thr Thr Thr Ala Gly Ser Gln Ser Gly Gly Asp
           275                 280                 285
   Ala Ser Ser Ala Leu Ala Trp Asn Ala Asp Val Ile His Gln Arg Gly
       290                 295                 300
   Gly Gln Thr Ile Asn Gly Thr Leu Arg Ile Asn Asn Thr Leu Thr Ile
   305                 310                 315                 320
   Ala Ser Gly Gly Ala Asn Ile Thr Gly Thr Val Asn Met Thr Gly Gly
                   325                 330                 335
   Tyr Ile Gln Gly Lys Arg Val Val Thr Gln Asn Glu Ile Asp Arg Thr
               340                 345                 350
   Ile Pro Val Gly Ala Ile Met Met Trp Ala Ala Asp Ser Leu Pro Ser
           355                 360                 365
   Asp Ala Trp Arg Phe Cys His Gly Gly Thr Val Ser Ala Ser Asp Cys
       370                 375                 380
   Pro Leu Tyr Ala Ser Arg Ile Gly Thr Arg Tyr Gly Gly Ser Ser Ser
   385                 390                 395                 400
   Asn Pro Gly Leu Pro Asp Met Arg Gly Leu Phe Val Arg Gly Ser Gly
                   405                 410                 415
   Arg Gly Ser His Leu Thr Asn Pro Asn Val Asn Gly Asn Asp Gln Phe
               420                 425                 430
   Gly Lys Pro Arg Leu Gly Val Gly Cys Thr Gly Gly Tyr Val Gly Glu
   435 440 445
   Val Gln Lys Gln Gln Met Ser Tyr His Lys His Ala Gly Gly Phe Gly
   450 455 460
   Glu Tyr Asp Asp Ser Gly Ala Phe Gly Asn Thr Arg Arg Ser Asn Phe
   465 470 475 480
   Val Gly Thr Arg Lys Gly Leu Asp Trp Asp Asn Arg Ser Tyr Phe Thr
   485 490 495
   Asn Asp Gly Tyr Glu Ile Asp Pro Ala Ser Gln Arg Asn Ser Arg Tyr
   500 505 510
   Thr Leu Asn Arg Pro Glu Leu Ile Gly Asn Glu Thr Arg Pro Trp Asn
   515 520 525
   Ile Ser Leu Asn Tyr Ile Ile Lys Val Lys Glu
   530 535

## Beschreibung

Die vorliegende Erfindung betrifft ein Verfahren zum Nachweis und zur Abreicherung von Endotoxinen aus einer Probe.

Endotoxin (ET) bezeichnet eine Familie von Lipopolysacchariden, die zusammen mit Proteinen und Phospholipiden die äußere Zellwand Gram-negativer Bakterien bilden. Endotoxine kommen ausschließlich in dieser Bakteriengruppe vor und spielen eine wichtige Rolle in der Organisation, Stabilität und Barrierefunktion der äußeren Membran. Zahlreiche Bakteriophagen nutzen Endotoxin bzw. allgemein Lipopolysaccharid zur spezifischen Erkennung ihrer Wirtsbakterien.

Alle Endotoxinvarianten bestehen aus einem Heteropolysaccharid, das kovalent an Lipid A, gebunden ist (Holst, O., 1999, Chemical structure of the core region of lipopolysaccharides. In: Endotoxin in health and disease (Brade, H., Morrison, D.C., Opal, S., Vogel, S. eds.), Marcel Dekker Inc. New York)). Lipid A verankert Endotoxin in der äußeren Bakterienmembran. Das Heteropolysaccharid, das aus einem Herzoligosaccharid und dem O-Antigen besteht, zeigt in die umgebende Lösung und bestimmt die serologische Identität des Bakteriums. Das O-Antigen besteht aus repetitiven Oligosaccharideinheiten, deren Zusammensetzung stammspezifisch ist (siehe hierzu Holst et al., supra). Charakteristische Bausteine des Herzoligosaccharids sind 2-Keto-3-desoxyoctonsäure (KDO) und L-Glycero-D-manno-heptose (Hep).

Der konservativste Teil von Endotoxin verschiedener Gattungen ist das Lipid A. Ähnlich konserviert wie Lipid A ist die innere Herzregion, die äußere Herzregion weist bereits eine höhere Variation auf Die innere Herzregion, KDO und Lipid A selbst tragen mehrere Phosphatgruppen als Substituenten und sind so für die negative Ladung von Endotoxin verantwortlich. Darüber hinaus können die Phosphatgruppen am Lipid A und der Herzregion variabel mit Arabinose, Ethanolamin und Phosphat substituiert sein. Einzelne Saccharidbausteine des O-Antigens sind acetyliert, sialysiert oder glycosyliert. Das O-Antigen variiert außerdem bezüglich der Anzahl repetitiver Einheiten, weshalb die Endotoxin-Population jedes Bakteriums eine gewisse Heterogenität aufweist (Palva E.T., Makela P.H., Lipopolysaccharide heterogeneity in Salmonella typhimurium analyzed by sodium dodecyl sulfate polyacrylamide gel electrophoresis. Eur J Biochem. 1980;107(1):137-43; Goldman R.C., Leive L., Heterogeneity of antigenic-side-chain length in lipopolysaccharide from Escherichia coli 0111 and Salmonella typhimurium LT2., Eur J Biochem. 1980;107(1):145-53).

Endotoxine sind Biomoleküle, die ohne entsprechende Vorsichtsmaßnahmen in praktisch allen wässrigen Lösungen vorzufinden sind. Endotoxine können bei Mensch und Tier zu Sepsis, einer starken Fehlreaktion des Immunsystems führen. Daher sind z.B. bei der Herstellung von Pharmaproteinen Verunreinigungen mit Endotoxin exakt nachzuweisen und in der Folge komplett zu entfernen. Endotoxin stellt ein Problem bei gentechnisch hergestellten Arzneimitteln, Gentherapeutika oder Substanzen dar, die in Mensch oder Tier (z.B. Tiermedizinische Behandlung oder bei Tierversuchen) injiziert werden. Doch nicht nur bei medizinischen, sondern auch bei Forschungsanwendungen, wie bei Transfektionsexperimenten von Säugerzellen kann eine Hemmung bzw. ein Senken der Transfektionseffizienz durch Endotoxin beobachtet werden.

Um Proteine im Rahmen von klinischen Studien einsetzen zu können, verlangen die europäische und die amerikanische Pharmacopeia, dass die Proteine bestimmte Grenzwerte an Endotoxinbelastung unterschreiten (z.B. Immunserum Globulin 0,91 EU/ml , dies entspricht 5 EU/kg Körpergewicht & Stunde (Dosis = EU/kg * h); EU = Endotoxin Unit; FDA (Food and Drug Administration): Guideline on Validation of LAL as End Product). Falls ein Medikament bzw. darin enthaltene Proteine eine zu hohe Endotoxinbelastung aufweisen, kann dies bis zum Tod des Probanden führen. Die fehlgeleitete Immunabwehr schädigt durch eine Überreaktion den Patienten. Dies kann zu Gewebeentzündungen, Blutdruckabfall, Herzrasen, Thrombose, Schock etc. fuhren. Bereits eine länger anhaltende Endotoxin-Exposition in Picogramm-Mengen kann zu chronischen Nebenwirkungen wie z.B. Immunschwächen, septischen Symptomen etc. führen. Im Rahmen der Substanzherstellung wird daher, insbesondere bei Prozessen unter "Good Manufacturing Practice" (GMP) Bedingungen, versucht, Endotoxin soweit wie möglich abzureichem. Allerdings ist die Endotoxin-Entfemung bei Proteinen, Polysacchariden und DNA problematisch. Gerade bei Proteinen gibt es große Probleme durch deren intrinsische Eigenschaften wie Ladungszustand oder Hydrophobizität, die eine Endotoxinentfernung nahezu verhindern bzw. zu großen Produktverlusten bei der Entfernungsprozedur führen können.

Derzeit sind nur drei Verfahren zum Endotoxin-Nachweis in biologischen Lösungen beschrieben, wobei nur die beiden ersten Verfahren von der FDA zugelassen sind. 1. "Rabbit Pyrogen Testing": Ein Verfahren, bei dem einem lebenden Kaninchen eine Endotoxin-Lösung injiziert und damit eine Immunreaktion ausgelöst wird. Diese Endotoxin-verursachte Immunantwort wird über die Entwicklung von Fieber nachgewiesen. 2. Deutlich besser standardisierbar ist der "Limulus Amoebocyte Lysate (LAL)" - Test, der derzeit am häufigsten verwendete Test (Bio Whittacker, Inc., Charles River, Inc., Associates of Cape Cod, Inc., alle USA). Bei diesem Verfahren wird die Verklumpung des Blutes des Pfeilschwanzkrebses (Limulus polyphemus) nach Endotoxin-Kontakt gemessen. 3. Eine weitere Möglichkeit ist der Einsatz eines speziellen Zellkultursystems (Sterogene Inc., USA), mit dem die Aktivierung von Monozyten über die Entstehung bestimmter Zytokine verfolgt wird.

Die beiden erstgenannten Verfahren sind jedoch sehr teuer (vgl. Konkurrenzvergleich Endotoxin-Nachweis) und durch den großen Bedarf an Versuchstieren bzw. an Blut des sehr seltenen Pfeilschwanzkrebses nicht zuletzt aus Tierschutzgründen bedenklich. Der LAL-Test kann zwar auch miniaturisiert und automatisiert werden, hat aber aufgrund geringer Stabilität der Komponenten massive Nachteile in der Anwendung. Eine einmal geöffnete LAL-Lösung muß direkt weiterverarbeitet und aufgebraucht werden, da die Komponenten innerhalb weniger Stunden aggregieren. Für alle Testverfahren ist geschultes Personal nötig und die Verfahren sind sehr störanfällig, weil z.B. das Immunsystem von Kaninchen auf die gleiche Endotoxindosis durchaus unterschiedlich reagieren kann. Das Zellkultur-Verfahren der Firma Sterogene ist, wie alle Zellkulturverfahren, ebenfalls sehr aufwändig und weist Probleme bei der Standardisierung auf.

Insgesamt kann festgestellt werden, dass es kein einfach handhabbares kostengünstiges Verfahren zum Endotoxinnachweis gibt und die derzeit eingesetzten Methoden eine Reihe von Nachteilen aufweisen. Es besteht daher der Bedarf für ein Verfahren, das diese Nachteile umgeht.

Zur Endotoxinabreicherung aus biologischen Lösungen allgemein gibt es eine Reihe von Verfahren. Insbesondere bei Proteinen gibt es allerdings bislang keine allgemein anwendbaren Standardverfahren. Die jeweils verwendeten Verfahren sind angepasst an die spezifischen Eigenschaften des jeweiligen Proteins und auf den entsprechenden Produktionsprozess des Proteins. Es gibt verschiedene Möglichkeiten zur Endotoxinabreicherung, wobei jedes dieser Verfahren spezifische Vor- und Nachteile aufweist.

Die Ultrafiltration (Petsch, D. & Anspach, F.B., 2000, J. Biotechnol. 76, 97-119 und Referenzen darin) wird für Endotoxin-Abreicherungen aus Wasser und Lösungen mit niedermolekularen Bestandteilen wie Salze, Zucker und Antibiotika verwendet, ist jedoch nicht für hochmolekulare Proteine oder DNA geeignet.

Die 2-Phasen-Extraktion (z.B. WO 0166718, Merck) soll wasserlösliche Proteine und DNA von Endotoxin trennen, bedingt jedoch Detergenzreste im gereinigten Produkt. Das Verfahren ist außerdem durch mehrmaliges Wiederholen der Reinigungsprozedur zeitaufwendig.

Ebenfalls wird für die Endotoxinabreicherung aus DNA und basischen Proteinen ein Anionenaustauscher (DEAE)-Verfahren verwendet (z.B. US 5990301, Qiagen; WO 9414837, Enzon), das jedoch eine niedrige Ionenstärke (<50 mM NaCl) voraussetzt und zu einer Protein Co-Adsorption bei sauren Proteinen führt.

Ein weiteres Verfahren zur Endotoxinabreicherung aus DNA und Proteinen (z.B. BSA, Myoglobin, gamma-Globulin, Cytochrom C) ist die Affinitäts-Adsorption (z. B. Polymyxin B, Histamine, Histidin, Polylysin) z.B. GB 2192633 (Hammersmith Hospital), die jedoch im Fall von Polymyxin B toxisch ist und bei niedrigen Ionenstärken zur Co-Adsorption von Proteinen führen kann.

Weiterhin wird die Immun-Affinitäts-Chromatographie eingesetzt, wobei die Spezifität für bestimmte Endotoxine nur über teure Antikörper (US 5179018, Centocor; WO 0008463, Bioserv) gegen Herz-Oligosaccharid erreicht werden kann.

Ferner wird das S3delta-Peptid (WO 0127289) des Faktors C (eines Bestandteils des LAL-Tests) (WO 9915676 beide: National University of Singapur) bei Proteinen (z.B. BSA, Chymotrypsinogen) verwendet, wobei jedoch dieses Verfahren eine geringe Effizienz bei hohen Ionenstärken besitzt und die hohen Herstellkosten (Produktion in Insekten-Zellkultur) hinzukommen. EP1399551, veroffenlicht unter WO 03/000888 ist Stand der Technik unter Artikel 54(3) EPÜ und veroffenlicht ein Verfahren zur Entfernung von Endotoxin aus einer Probe mittels Bakteriophagenproteine.

In der Anwendung in der pharmazeutischen Industrie befinden sich für Proteinlösungen, angepasst an die Eigenschaften der Zielproteine im wesentlichen drei Verfahren:
- Anionenaustauscherchromatographie
- Reversed-Phase Chromatographie; Diese hat den Nachteil, dass sie nicht für alle Proteine gleichermaßen geeignet, - insbesondere bei hydrophoben Proteinen problematisch ist. Darüberhinaus ist dieses Verfahren sehr zeitintensiv.
- RemTox (Fa. Millipore): Dieses Verfahren hat den Nachteil, das neben einer sehr langen Inkubationsdauer, der unspezifische Bindungsanteil hoch ist, und die Proteinwiederfindung oftmals nicht ausreichend ist.

Eine grobe Endotoxin-Abreicherung von Proteinen auf einen Wert bis zu 10 EU/ml ist mit den bestehenden Verfahren in vielen Fällen möglich. Die verbleibende Konzentration an Endotoxin wirkt jedoch immer noch toxisch. Eine weitere Abreicherung (=Feinreinigung) ist daher geboten bzw. abhängig von der Dosis des Proteins in der medizinischen Anwendung, von der Europäischen Pharmacopeia (z.B. 5 EU/kg Körpergewicht und Stunde in intravenösen Anwendungen) und der FDA verbindlich vorgeschrieben. Allerdings ist diese Feinreinigung mit vorhandenen Methoden oft nicht zufriedenstellend gewährleistet. Die marktgängigen Verfahren weisen hier erhebliche Nachteile auf und sind bei bestimmten Proteinen oft nicht, oder nur unter erheblichen Verlusten des Zielproteins, anwendbar.

Daher liegt der Erfindung die Aufgabe zu Grunde, ein Verfahren bereitzustellen, das Endotoxine in Proben nachweisen kann. Der Erfindung liegt ferner die Aufgabe zu Grunde, ein Verfahren bereitzustellen, mit dem Endotoxine aus wässrigen Lösungen entfernt werden können.

Die Aufgaben werden durch den in den Patentansprüchen definierten Gegenstand gelöst.

Die nachfolgenden Figuren erläutern die Erfindung.

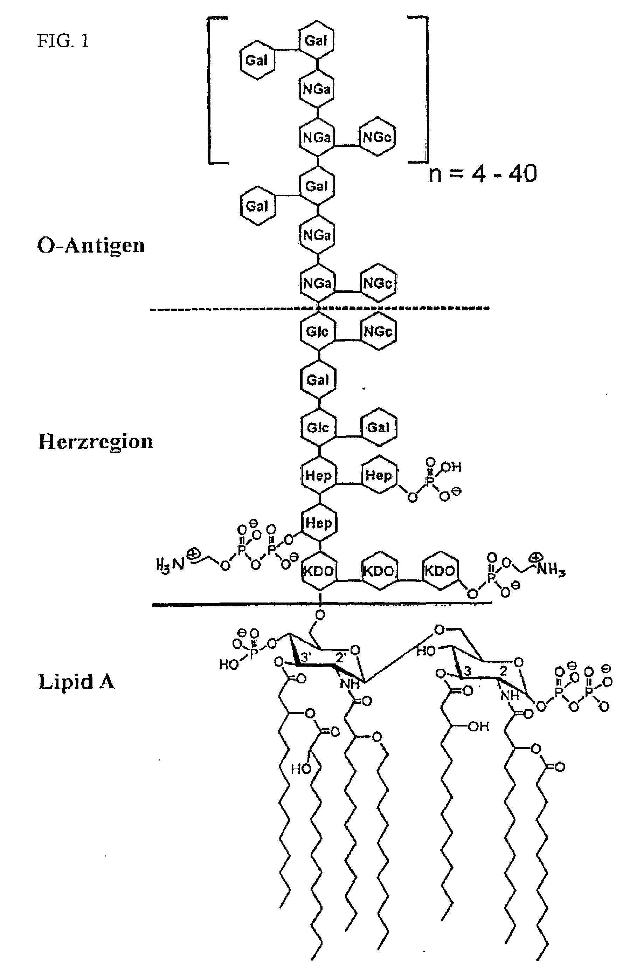

Fig. 1 zeigt eine schematische Übersicht der chemischen Struktur von Endotoxin aus E. coli 0111:B4. Hep = L-Glycero-D-manno-heptose; Gal = Galactose; Glc = Glucose; KDO = 2-Keto-3-desoxyoctonsäure; NGa = N-Acetyl-galactosamin; NGc = N-Acetylglucosamin.

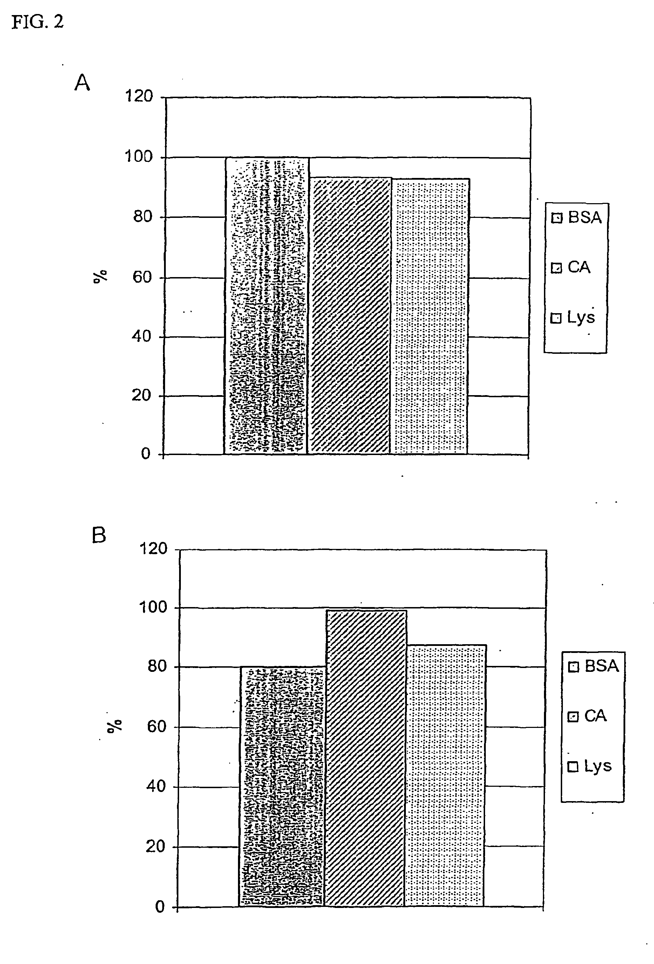

Figur 2 zeigt die Ergebnisse von Versuchen mit Chromatographiesäulen, die über Sulfhydrylreste immobilisiertes NStrepS3Cp12 tragen. (A) Endotoxinentfernung aus Proteinlösungen: Rinderserumalbumin (BSA), Carbonanhydrase (CA) und Lysozym (Lys) wurden 1 h auf der Säule inkubiert und anschließend mit Puffer eluiert. Die Endotoxinkonzentration vor und nach der Säule wurden mit dem LAL-Test gemessen und daraus die prozentuale Abnahme berechnet. (B) Proteinwiederfindung: Die Proteinkonzentrationen der Ausgangslösungen und der Fraktionen nach der Säule wurden durch Absorptionsmessung bei 280 nm bestimmt und daraus die prozentuale Proteinwiederfindung ermittelt.

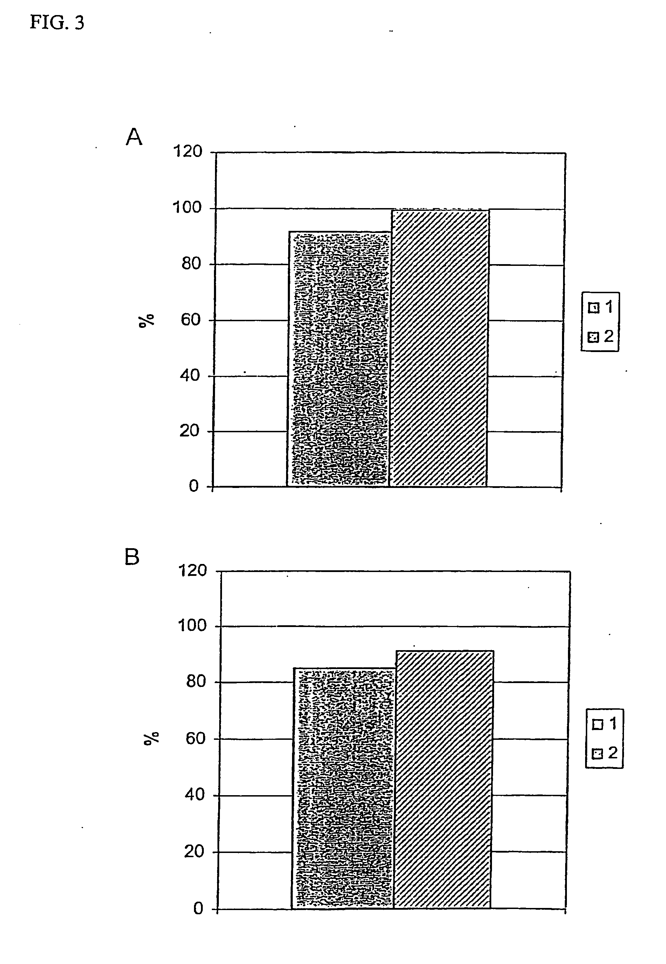

Figur 3 zeigt die Endotoxinentfernung aus einer Lysozymlösung über Chromatographiesäulen mit "ungerichtet" (1) und _{"}gerichtet" (2) immobilisiertem p12. In beiden Fällen wurde an NHS-aktivierte Säulen p12 S3C gebunden. Die "ungerichtete" Immobilisierung erfolgte über primäre Aminoreste von p12S3C, die durch Reaktion mit den NHS-Gruppen kovalente Verbindungen mit der Trägersubstanz eingehen. Eine "gerichtete" Verknüpfung von p12S3C über ein N-terminales Cystein wird durch Diaminoethan und SIA (N-succinimidyl-iodoacetat) erreicht. (A) prozentuale Endotoxinentfemung. (B) Proteinwiederfinung.

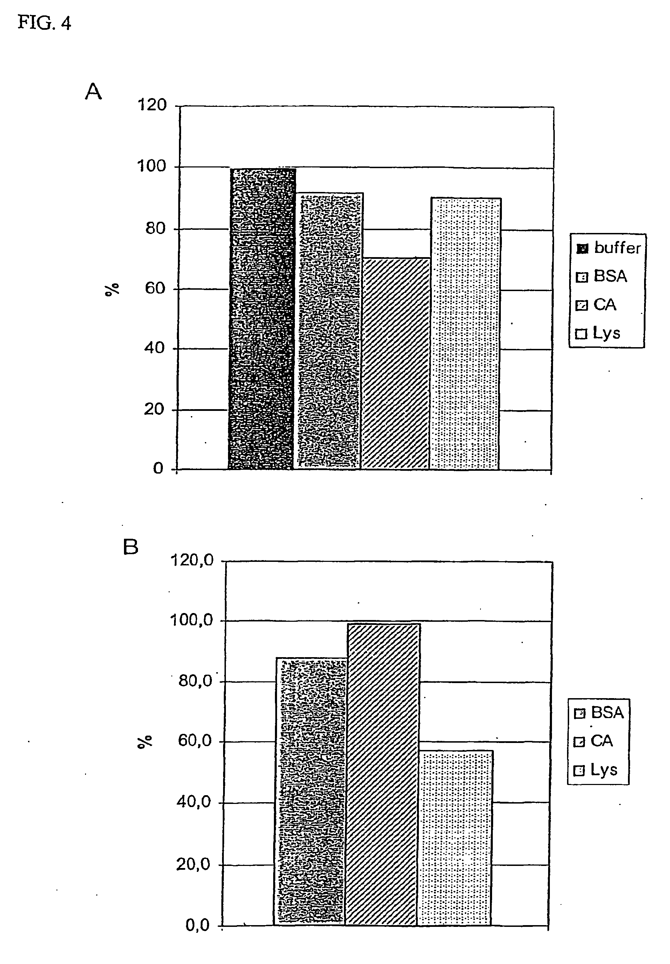

Figur 4 zeigt die Ergebnisse von Versuchen mit biotinyliertem p12, das über Streptavidin an magnetische Beads gebunden wurde. (A) Die Endotoxinabreicherung aus Puffer (20 mM Hepes, 150 mM NaCl, pH 7.5) und Proteinlösungen wurde mittels LAL-Test bestimmt. (B) Für die Proteinlösungen wurde die Proteinwiederfindung durch Absorptionsmessungen ermittelt. Die Abtrennung der Beads von der Lösung erfolgte mit Hilfe eines Magnetseparators. BSA: RinderSerumalbumin. CA: Carbonanhydrase. Lys: Lysozym.

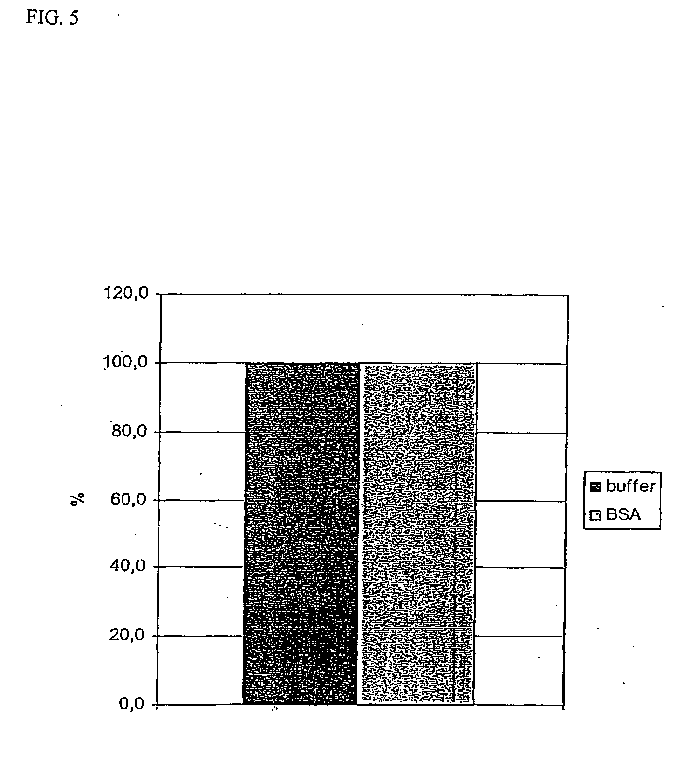

Figur 5 zeigt die Ergebnisse der Endotoxinentfernung mit p12, das über Biotin-Streptavidin Wechselwirkungen auf Agarose-Beads immobilisiert wurde. Die Abtrennung des immobilisierten p 12 erfolgte durch Zentrifugation. Die Endotoxinentfernung aus Puffer (20 mM Tris, 150 mM NaCl, pH 8.0) und BSA-Lösungen wurde anhand der Endotoxinkonzentrationen von Ausgangslösung und Überstand bestimmt.

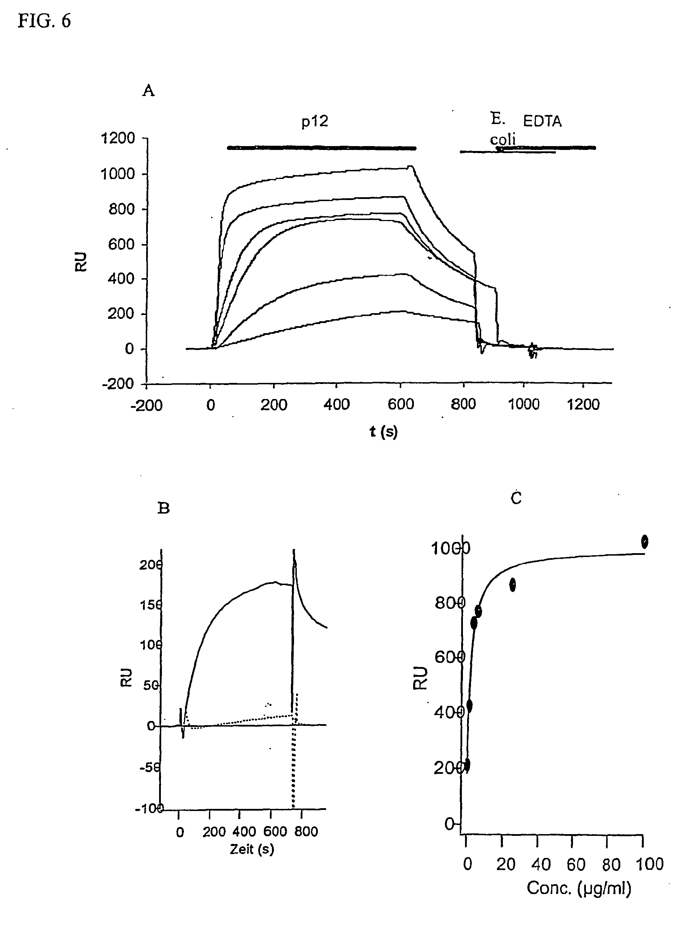

Figur 6 zeigt Ergebnisse von Oberflächen-Plasmon-Resonanz Messungen. (A) Resonanzkurven, die als Antwort auf Injektion von verschiedenen (je in µg/ml: 100; 25; 6,25; 4; 1,56; 0,4) p12-Konzentrationen (__) gemessen wurden. Die Bindung erfolgt an Endotoxin von E. coli D21f1, das auf einem hydrophoben HPA-Chip immobilisiert wurde. Die Injektion von p12 und EDTA (5 mM) wird durch Balken über den Kurven markiert. Puffer: 20 mM Tris, 150 mM NaCl, pH 8.0. (B) Gleichgewichtsresonanzwerte für die Bindung von p12 an immobilisiertes Endotoxin wurden etwa 600 s nach Beginn der p12 Injektion gemessen und gegen die dazugehörigen p12-Konzentration aufgetragen. Die durchgezogene Linie zeigt einen Fit der Langmuirsche Adsorptionsisotherme (RU = RUₘₐₓ*[p12]/([p12]+K_{d})) an die Daten. (C) Bindung von E. coli an biotinyliertes p12, das auf Streptavidin-Chips immobilisiert wurde. E. coli D21e8 (____), dessen innerere Herz-Region vollständig ist, an p12. Dagegen bindet E. coli D21f2 (----), der eine stark verkürzte Herz-Region besitzt, bindet nicht an p12. Die Messungen wurden in PBS durchgerührt.

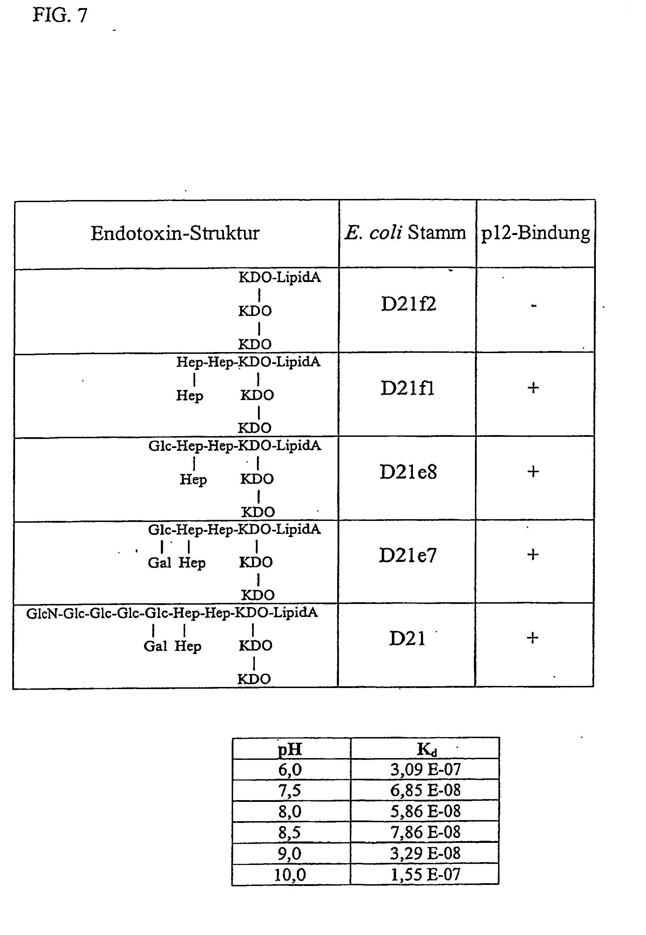

Figur 7 zeigt schematisch die Struktur der Endotoxin-Herzregion verschiedener E.coli-Mutanten.

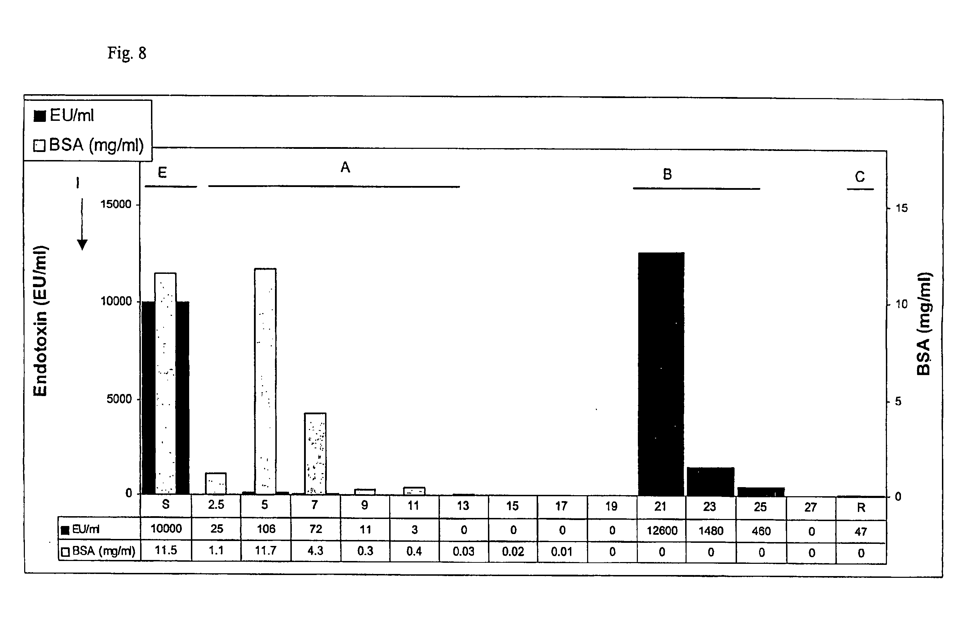

Figur 8 zeigt schematisch das Ergebnis einer Endotoxin-Abreicherung mittels Chromatographiesäulen-Durchflussverfahren. E bedeutet Äquilibrierungspuffer (20 mM Hepes, 150 mM NaCl, 0.1 mM CaCl₂, pH 7.5), A bedeutet Waschpuffer A (20 mM Hepes, 150 mM NaCl, 0.1 mM CaCl₂, pH 7.5), B bedeutet Elutionspuffer B (20 mM Hepes, 150 mM NaCl, 2 mM EDTA, pH 7.5), C bedeutet Regenerationspuffer C (20 mM Hepes, 150 mM NaCl, 2 mM EDTA, 0.005 % NaDOC, pH 7.5), S bedeutet Konzentration von Protein und Endotoxin in der Ausgangslösung. BSA bedeutet Rinderserumalbumin. EU bedeutet Endotoxin Units (Endotoxin Einheiten). Nach Injektion (I) von 4 ml der Ausgangslösung (S) wurde mit 15 ml Waschpuffer nachgespült und der Durchlauf fraktioniert (je 2.5 ml während des Auftrags, je 2 ml während des Waschens). Anschliessend wurde die Säule mit den Puffern B und C regeneriert und der Auslauf ebenfalls in Fraktionen (je 2 ml) gesammelt. Wie in der Figur ersichtlich, war das BSA in den ersten 3-5 Fraktionen nach der Injektion zu finden. Der Gehalt an Endotoxin in diesen Fraktionen war um den Faktor 100 niedriger als in der Ausgangslösung. Das an die Säule gebundene Endotoxin wurde dann mit den Puffern B und C von der Säule gewaschen.

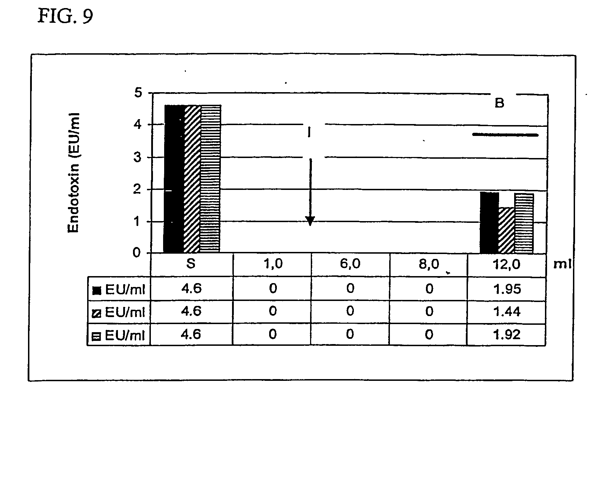

Figur 9 zeigt schematisch die Ergebnisse der Endotoxin Entfernung aus gering verunreinigter Pufferlösung (5 EU/ml) im Durchflussverfahren. p12 wurde ungerichtet auf NHS-aktivierter Sepharose 4 FastFlow (Amersham Biosciences, Uppsala, Schweden) immobilisiert (8 mg p12/ 1 ml Sepharose) und 3 Säulen mit je 2 ml Säulenvolumen gegossen. Das Experiment wurde parallel auf 3 Säulen durchgeführt. Vor dem Auftrag der Probe wurde jeweils 1 ml Äquilibrierungspuffer (20 mM Hepes, 150 mM NaCl, 0.1 mM CaCl₂, pH 7.5) gesammelt, danach die Probe (S: Endotoxin aus E. coli O55:B5 in Äquilibrierungspuffer, 4.6 EU/ml) injiziert (I) und Fraktionen von 5 ml und 2 ml gesammelt. Die Regeneration der Säule erfolgte durch Zugabe von 4 ml Regenerationspuffer (B: 20 mM Hepes, 150 mM NaCl, 2 mM EDTA, 0.005 % NaDOC, pH 7.5). Die Endotoxin Konzentration wurde mittels LAL-Test bestimmt (kinetisch chromogener LAL-Test, Charles River Inc.). Die Endotoxin Verunreinigungen konnten in allen drei Experimenten vollständig entfernt werden, d.h. die Endotoxin Konzentration im Durchlauf lag unter der Nachweisgrenze (<0.005 EU/ml).

Der Begriff "Endotoxinabreicherung" wie hier verwendet bedeutet vollständige oder teilweise Entfernung von Endotoxin aus Probenmaterial.

Der Begriff "Endotoxin" wie hier verwendet bezeichnet bakterielles Lipopolysaccharid, das Bestandteil der äußeren Membran gram-negativer Bakterien ist.

Der Begriff "Bakteriophagenschwanzprotein" wie hier verwendet bezeichnet solche Proteine, die in Bakteriophagen vorkommen und Bestandteile von Zellmembranen binden können. Üblicherweise sind diese Proteine im Bakteriophagenschwanz lokalisiert, können jedoch auch auf dem Bakteriophagenkopf oder bei Bakteriophagen ohne Schwanz auf der normalen Bakterienhülle lokalisiert sein. Die von dem Bakteriophagenschwanzprotein gebundenen Zellbestandteile erkennen insbesondere Endotoxine.

Der Begriff "unspezifische Immobilisierung" oder "ungerichtete Immobilisierung" wie hier verwendet bedeutet, dass die Kopplung eines Proteins an eine Matrix über Proteinreste (z.B. primäre Amine) erfolgt, die über die gesamte Proteinoberfläche verteilt sind. Die Auswahl der für die Kopplung des einzelnen Proteinmoleküls verwendeten Gruppe ist zufällig.

Der Begriff "gerichtete Immobilisierung" wie hier verwendet bedeutet, dass die Kopplung über Aminosäurereste oder andere Reste (z.B. Glykosyslierungen des Proteins) erfolgt, deren Position im Protein (z. B. N- oder C-terminal) bekannt ist. Die Auswahl dieser Gruppen für die Kopplung erfolgt durch die Auswahl geeigneter Reaktionspartner/Linker, die bevorzugt mit diesen Resten reagieren (z.B. Kopplung von Sulfhydrylresten an Iodoacetatreste; Iodoacetat reagiert tausendmal schneller mit Sulfhydrylresten als mit Aminoresten).

Die vorliegende Erfindung betrifft ein Verfahren zum Nachweis von Endotoxin, umfassend die Schritte:
a) Inkubieren einer Probe mit einem Bakteriophagenschwanzprotein,
b) Nachweis von an Bakteriophagenschwanzproteine gebundenes Endotoxin.

Vorzugsweise betrifft die Erfindung ein Verfahren, bei dem der Nachweis mittels spektroskopischer Verfahren, z.B. Fluoreszenzemission, Fluoreszenzpolarisation, Absorption oder Circulardichroismus, oder mittels Kapazitätsmessung, z.B. elektrische Signale, oder indirekt mittels Kompetitionsnachweis durchgeführt wird.

Gegebenenfalls wird nach Schritt a) und vor Schritt b) ein zusätzlicher Schritt a') Abtrennung von Bakteriophagenschwanzprotein-Endotoxin-Komplex von der Probe eingeführt.

Die vorliegende Erfindung betrifft ferner ein Verfahren zur Entfernung von Endotoxin aus einer Probe, umfassend die Schritte:
a) Inkubation oder in Kontakt bringen einer Probe mit Bakteriophagenschwanzproteinen, die unspezifisch oder gerichtet, an einem festen Träger immobilisiert sind,
b) Trennen des Bakteriophagenschwanzprotein-Endotoxin-Komplexes von der Probe.

Vorzugsweise wird vor der Inkubation die Ionenzusammensetzung der zweiwertigen Ionen z.B. Ca²⁺, Mg²⁺ und/oder der pH-Wert eingestellt, um eine optimale Endotoxin-Bakteriophagenschwanzprotein-Bindung zu erhalten. Ferner bevorzugt wird bei oder nach der Inkubation eine "Demaskierung" des gebundenen Endotoxins durch Zugabe von Detergentien und/oder Salzen, z.B. Tween, Triton, NaCl oder Ammoniumsulfat, oder anderer Substanzen, z.B. Chitosan, Zucker oder Lipide, die ein Ablösen der Endotoxine von z.B. Proteinen oder Nukleinsäuren beschleunigen.

Das Bakteriophagenschwanzprotein kann ein natürlicherweise vorkommendes oder ein molekularbiologisch oder biochemisch modifiziertes sein. Das Bakteriophagenschwanzprotein kann aus verschiedenen Gründen gentechnisch und/oder biochemisch modifiziert sein. Für die erfindungsgemäßen Verfahren können jedoch nicht nur die natürlicherweise vorkommenden Bakteriophagenschwanzproteine verwendet werden, sondern auch deren Varianten. Varianten bedeutet im Sinne der vorliegenden Erfindung, dass die Bakteriophagenschwanzproteine eine veränderte Aminosäuresequenz aufweisen. Diese können durch Screening der natürlich auftretenden Varianten, oder durch Zufalls-Mutagenese oder gezielte Mutagenese, aber auch durch chemische Modifikation erhalten werden. Die für die erfindungsgemäßen Verfahren verwendeten Bakteriophagenschwanzproteine können durch eine gezielte oder zufällige Mutagenese in ihrer Spezifität bzw. ihren Bindungseigenschaften an Trägerstrukturen angepaßt werden. Diese Bindung an die Träger kann fest, z.B. kovalent oder über eine spezifische oder unspezifische Biotinylierung erfolgen, aber auch reversibel z.B. über eine reduzierbare Disulfidbrücke erfolgen. Ferner kann durch eine Modifikation die Stabilität erhöht werden. Durch die molekularbiologische oder chemische Mutagenese werden Mutationen eingeführt, die Aminosäureadditionen, -deletionen, -substitutionen oder chemische Modifikationen sein können. Diese Mutationen können eine Veränderung der Aminosäuresequenz in der Bindungsregion der Bakteriophagenschwanzproteine bewirken, mit dem Ziel, Spezifität und Bindungsaffinität an Testbedürfnisse anzupassen, z.B. die Bindung der Endotoxine an die Bakteriophagenschwanzproteine zu erhöhen oder irreversibel zu machen, um den Nachweis oder die Abreicherung zu verbessern. Darüber hinaus kann eine gentechnische oder biochemische Modifikation der Phagenproteine durchgeführt werden, mit dem Ziel, die gegebenenfalls vorhandene enzymatische Aktivität auszuschalten, um dadurch die Bindung zu verbessern oder irreversibel zu machen. Weiterhin kann eine gentechnische oder chemische Modifikation der Phagenproteine durchgeführt werden, um die vorhandenen physikalischen Eigenschaften des Proteins wie Löslichkeit Thermostabilität usw. im Sinne des erfindungsgemäßen Verfahrens anzupassen.

Arbeiten zur Aufklärung der dreidimensionalen Struktur von T4 p12 hatten gezeigt, dass bei erhöhter Temperatur proteolytische Fragmente von 33 kDa und 45 kDa erzeugt werden können, die N- und C-terminal (33 kDa) bzw. nur N-terminal (45 kDa) verkürzt sind. Im Gegensatz zu dem 33kDa Fragment ist das 45kDa Fragment noch in der Lage an Bakterien zu binden. Demzufolge ist der C-Terminus an der Zellbindung beteiligt.

Die Modifikation kann ferner insbesondere den Zweck haben, einen direkten Nachweis z.B. mittels Messung der Tryptophanfluoreszenz zu ermöglichen. Beispielsweise besitzt P12 fünf Tryptophan-Reste. Das Fluoreszenzspektrum des nativen Proteins deutet darauf hin, dass diese Reste weitestgehend lösungsmittel-unzugänglich sind. Aus einer Vielzahl von wissenschaftlichen Arbeiten ist bekannt, dass fast immer aromatische Aminosäuren an der Bindung von Zuckerresten, wie sie auch in Endotoxin vorkommen, beteiligt sind. Die Bindung der Zuckerreste an Proteine kann durch einen Quench der Trypthophanfluoreszenz, bzw. gegebenenfalls auch zusätzlich durch eine Veränderung des Fluoreszenzmaximums verfolgt werden. Eigene Arbeiten lassen vermuten, dass die ungünstige Verteilung der Fluorophore des natürlichen p12 eine Ausnutzung der Fluoreszenz-Eigenschaften von p12 zur Bindungsmessung verhindert. Die Fluoreszeneigenschaften von p12 werden durch die fünf Tryptophanreste dominiert, deren Fluoreszenz durch die Zugabe von Endotoxin nicht messbar verändert wird. Diese Daten lassen erwarten, dass eher Tyrosinreste als Tryptophanreste an der Bindung beteiligt sind, deren Signaländerung vor dem hohen Tryptophan-Hintergrund nicht sichtbar gemacht werden kann. Auf der Basis der Proteolyseergebnisse kommen sechs Tyrosine am C-Terminus von p12 für den Endotoxin-Nachweiskit in Frage, die entsprechend "sichtbar" gemacht werden können. Durch einen selektiven molekularbiologischen Austausch der fünf Tryptophan-Reste gegen Tyrosine werden in einem ersten Schritt die spektroskopischen Eigenschaften so gezielt verändert, dass die Endotoxin-Bindung per Fluoreszenzsignaländerung eines einzelnen Tryptophanrestes messbar ist. Anschließend wird durch einen gezielten Austausch von jeweils einem der sechs Tyrosine im C-terminalen Bereich gegen einen Tryptophanrest die Intensität des messbaren Signals signifikant erhöht, um für die Entwicklung eines Endotoxin-Nachweiskits attraktive Signalunterschiede zu erhalten.

Welche Bakteriophagenschwanzproteine verwendet werden, hängt davon ab, welche Endotoxine nachgewiesen oder abgereinigt werden sollen. Bereits jetzt steht eine große Zahl bekannter Bakteriophagen für einen Großteil der bisher beschriebenen Bakterien zur Verfügung und kann für die erfindungsgemäßen Verfahren verwendet werden. Die Phagen und die entsprechenden Wirtsbakterien sind u.a. bei folgenden Stammsammlungen erhältlich: ATCC (USA), DSMZ (Deutschland), UKNCC (Großbritannien), NCCB (Niederlande) und MAFF (Japan).

Vorzugsweise stammen die Bakteriophagenschwanzproteine für die erfindungsgemäßen Verfahren von Bakteriophagen, deren Wirtsbakterien medizinisch oder biotechnologisch relevante Bedeutung haben, wie z.B. E. coli, das bei der Produktion rekombinanter Proteine oder von Nukleinsäuren für die Gentheraphie verwendet wird. Besonders bevorzugt sind Bakteriophagenschwanzproteine, die stark konservierte Bereiche von Endotoxin binden, wie z.B. die Herzregion oder Lipid A. Insbesondere bevorzugt sind p12 und p12-ähnliche Bakteriophagenschwanzproteine Bei einer Kombination von Endotoxin-Verunreinigungen aus verschiedenen Wirtsbakterien kann eine Kombination der entsprechenden Endotoxinerkennenden Bakteriophagenschwanzproteine eingesetzt werden.

Der Nachweis oder die Abreicherung von Endotoxin in oder aus einer Probe erfolgt über die Bindung von Endotoxin an die Bakteriophagenschwanzproteine. Diese Bindung kann z.B. durch direkte Messung mittels spektroskopischer Verfahren, z.B. über Fluoreszenzemission, Fluoreszenzpolarisation, Absorption oder Circulardichroismus nachgewiesen werden. Darüber hinaus kann die Bindung durch elektrische Signale, z.B. eine Kapazitätsmessung sichtbar gemacht werden. Weiterhin kann die Bindung von Endotoxin an die Bakteriophagenschwanzproteine auch indirekt über Verdrängungsexperimente nachgewiesen werden.

Für den erfindungsgemäßen Nachweis können die Bakteriophagenschwanzproteine bei Bedarf einer Abtrennung der Bakteriophagenschwanzprotein-Endotoxin-Komplexe von der Probe auf geeigneten Trägerstrukturen, z.B. Magnetpartikeln, Agarosepartikeln, Mikrotiterplatten, Filtermaterialien oder Durchflußzellkammem, gekoppelt werden (indirekter Nachweis). Die Trägerstrukturen können z.B. aus Polystyrol, Polypropylen, Polycarbonat, PMMA, Celluloseacetat, Nitrozellulose, Glas, Silizium oder Agarose bestehen. Die Kopplung kann z.B. durch Adsorption oder kovalente Bindung erreicht werden.

Für das erfindungsgemäße Abreicherungsverfahren sind die Bakteriophagenschwanzproteine an feste Träger gekoppelt. Die festen Träger können Materialien für Chromatographiesäulen (z.B.

Sepharosematerialien), Filtrationsmedien, Glaspartikel, Magnetpartikel, Zentrifugations- oder Sedimentationsmaterialien (z.B. Agarosepartikel) sein.

Wichtig hierbei ist eine funktionelle Kopplung, d.h. Bakteriophagenschwanzproteine verfügen trotz Bindung an das Trägermaterial über für Endotoxin zugängliche Strukturen. Die Kopplung der Bakteriophagenschwanzproteine kann unspezifisch, oder aber bevorzugt gerichtet, über z.B. eine selektive Biotinylierung, oder gekoppelt über einen Spacer oder Linker erfolgen.

Dazu können die Bakteriophagenschwanzproteine mit niedermolekularen Substanzen z.B. Biotin verknüpft sein, um über diese niedermolekularen Substanzen an Polypeptide z. B. Streptavidin zu binden, die ihrerseits auf dem Träger immobilisiert wurden. Statt Biotin kann ferner der sogenannte Strep-Tag (Skerra, A. & Schmidt, T. G. M. Biomolecular Engineering 16 (1999), 79-86) verwendet werden, der eine kurze Aminosäuresequenz ist und an Streptavidin bindet. Ferner kann der His-Tag verwendet werden, der über zweiwertige Ionen (Zink oder Nickel) oder einen für ihn spezifischen Antikörper (Qiagen GmbH, Hilden) an ein Trägermaterial binden kann. Der Strep-Tag sowie der His-Tag wird vorzugsweise über DNA-Rekombinationstechnologie an die rekombinant hergestellten Bakteriophagenproteine gebunden. Diese Kopplung kann gerichtet, z.B. am N- oder C-Terminus oder ungerichtet erfolgen. Die gerichtete Kopplung erfolgt über eine geeignete, reaktive natürlicherweise bei Phagenproteinen nicht häufig oberflächenexponierte Aminosäure wie Cystein, das an geeigneter Stelle gezielt eingeführt wurde. Da Phagenschwanzproteine im Cytoplasma synthetisiert werden, ist nicht mit Disulfidbrücken zu rechnen. Vorzugsweise kann auch über andere Aminosäuren direkt, oder wie auch bei Cystein über einen "Spacer" oder "CrossLinker" (Monofunktionell oder bifunktionell) indirekt gekoppelt werden.

Bei der Cysteinkopplung sind alle bifunktionellen Crosslinker mit NH- und SH-reaktiven Gruppen, mit und ohne Zwischenspacer, z.B. 11-Malcimidoundecanoic acid sulfo-NHS oder Succinimidyl-4-[N-maleimidomethyl]-cyclohexane-1-carboxy-[6-amido]caproate möglich. Sofern keine Spacer vorhanden sind, können 8-12 C-Atom-Spacer mit endständiger NH-Gruppe eingefügt werden. Vorzugsweise erfolgt die Cysteinkopplung über eine spezifische Biotinylierung des Cysteins durch z.B. EZ-Link-PEO-Maleimide activated Biotin (Pierce).

Zweiwertige Ionen, wie z.B. Ca²⁺ oder Mg²⁺ sind für eine Bindung von Endotoxinen an Phagenproteine wie p12 wichtig. Durch Zugabe von geeigneten Chelatoren, wie z.B. EDTA oder EGTA, kann diese Bindung jedoch gelöst werden. Bevorzugt für die Bindung sind Ca²⁺⁻Konzentrationen im Bereich von etwa 0,1 µM bis etwa 100 mM, besonders bevorzugt im Bereich von etwa 0,1 µM bis etwa 10 mM, insbesondere bevorzugt im Bereich von etwa 0,1 µM bis etwa 1 mM und ferner insbesondere bevorzugt im Bereich von etwa 10 µM bis 1 mM. Erniedrigt man die Konzentration zweiwertiger Ionen durch Zugabe von 1 mM EDTA unter 100 nM, so wird die Bindung von Endotoxin an p12 gelöst. Mg²⁺-Konzentrationen über 10 mM verschlechtern die Bindung von Endotoxin an p12, was sich in einer Erhöhung der Dissoziationskonstante bemerkbar macht. Ohne Zugabe von Mg²⁺ ergibt sich ein K_{d}-Wert von 50 nM und in einem Puffer mit 10 mM Mg²⁺ wurde ein K_{d}-Wert von 1 µM gemessen. Zink zeigte eine noch stärker hemmende Wirkung. 1 mM Zn erhöht den K_{d}-Wert auf 10 µM. Eine Einstellung der Konzentration zweiwertiger oder anderer Ionen (z.B.: Cu²⁺, A1³⁺, Zn²⁺, Fe²⁺, Ca²⁺, Ba²⁺, Mg²⁺, Cd²⁺) auf einen für die Bindung optimalen Bereich kann durch Substanzen, wie HEDTA, NTA bzw. allgemein Chelatoren/Puffer (ADA: N-[2-Acetamido]-2-iminodiacetic acid; 5-AMP: Adenosin-5'-Monophosphat; ADP: Adenosin-5'-Diphosphat; ATP: Adenosin-5'-Triphosphat; Bapta: 1,2-bis(2-Aminophenoxy)ethane-N,N,N',N'-tetraacetic acid; Citrat: Zitronensäure; EDTA: Ethylendiamintetraacetic acid; EGTA: Ethleneglycol-bis(β-aminoethyl Ether) N,N,N',N'-Tetraacetic acid; HEDTA: N-hydroxyethylethylenediaminetriacetic acid; NTA: Nitrilotiracetic acid; SO₄. Sulfat) erfolgen, die als Puffer für zweiwertige Ionen benutzt werden können.

Die erfindungsgemäßen Verfahren können daher ferner Waschschritte umfassen. Je nachdem, ob ein direkter oder indirekter Nachweis oder die Abreicherung eine Abtrennung von Probe und Bakteriophagenschwanzprotein nötig macht, können Waschschritte eingebaut werden. Da Ca2+ oder andere Metallionen (z.B. Mg2+) essentiell für die Bindung sind, kann die Bindung von Endotoxin an z.B. p12 durch geeignete Waschschritte gelöst werden. Je nach Ziel, ob Endotoxin auf dem Bakteriophagenschwanzprotein, z.B. p12 gebunden bleiben soll, wird mit EDTA-freiem Puffer gewaschen, wenn die Bindung gelöst werden soll mit EDTA-haltigem Puffer, wobei die EDTA-Konzentrationen im Bereich von mindestens 0,05 mM bis mehr als 10 mM, vorzugsweise im Bereich von 2 mM bis 5 mM liegt.

Die Abtrennung erfolgt nach Inkubation der Probe mit dem entsprechenden mit Bakteriophagenschwanzproteinen gekoppelten Trägermaterial für etwa 5-60 min oder etwa 30-180min oder bei Bedarf auch über Nacht. Dazu wird die Probe z.B. aus der Chromatographiesäule eluiert, oder filtriert, oder die entsprechenden Partikel abzentrifugiert oder absedimentiert, bzw. durch Anlegen eines Magnetfeldes magnetisch separiert. Die Abtrennung in dem hier beschriebenen Batch-Verfahren, d.h. mit Vorinkubation von Probe und mit den entsprechenden Bakteriophagenschwanzproteinen gekoppelten Trägermaterialen, kann insbesondere bei sehr niedrigen Endotoxinkonzentrationen sinnvoll sein.

Die Abreicherung von Endotoxinen über Chromatographiesäulen kann aber auch im reinen Durchflussverfahren erfolgen. Die Probe kann dazu auf die Säule aufgetragen werden, die ein Trägermaterial mit daran gekoppelten Bakteriophagenschwanzproteinen enthält. Die Flussrate ist abhängig von Volumen und Geometrie der Säule. Die Flussrate ist ferner abhängig von Volumen und Endotoxingehalt der Probe, um durch eine möglichst lange Kontaktzeit zwischen Säule und Endotoxin auch bei niedrigen Endotoxinkonzentrationen eine effiziente Abreicherung zu erzielen. Die Kontaktzeit ist dabei die Zeit, die die Probe vom Auftragen auf die Säule bis zum Herausfließen benötigt.

Der Abtrennschritt kann z.B. im Abreicherungsverfahren zur Regenerierung der Bakteriophagenschwanzproteine benutzt werden, die an den festen Träger gekopplelt sind. Dadurch kann der feste Träger, z.B. eine Matrix in einer Chromatographiesäule wiederverwendet werden. Die Regenerierung erfolgt durch Entfernen des gebundenen Endotoxins durch einen geeigneten Regenerierungspuffer enthaltend EDTA oder einen entsprechenden Chelator. Bei EDTA wird eine Konzentration von größer 2 mM EDTA bevorzugt, inbesondere größer 10 mM EDTA.

Da ionische Wechselwirkungen grundsätzlich immer durch Veränderungen der Ionenstärke beeinflussbar sind, können auch Erhöhungen oder Erniedrigungen anderer Salze in Lösung, wie z.B. NaCl oder KCl, die Bindung von Endotoxin an die Bakteriophagenschwanzproteine beeinflussen.

Um die Bindung im Nachweisverfahren direkt oder indirekt sichtbar zu machen, kann auch das Protein molekularbiologisch oder biochemisch verändert werden, um die Messung zu ermöglichen, bzw. zu verbessern. Um eine Bindung von Endotoxin z.B. an p12 direkt sichtbar zu machen, kann ein molekularbiologischer Austausch von Tyrosinresten gegen Tryptophan durchgeführt werden. Für eine Reduktion des Signalhintergrundes kann es dabei nötig sein, die ursprünglich enthaltenen Tryptophane gegen Tyrosine auszutauschen.Um auch in proteinhaltigen Lösungen messen zu können, kann p12 nach Tryptophan-Einführung zusätzlich chemisch modifiziert werden. Dabei werden Tryptophanreste durch Koshland-Reagenz (2-Hydroxy-5-nitrobenzylbromid) hinsichtlich ihrer spektrokopischen Eigenschaften verändert. Bei Verdrängungsexperimenten kann markiertes, z.B. fluoreszenzmarkiertes Endotoxin (z.B. Sigma) durch in der Probe befindliches Endotoxin z.B. von p12 verdrängt und die Konzentration von freiern fluoreszierendem Endotoxin bestimmt werden.

Mit dem erfindungsgemäßen Verfahren kann Endotoxin aus und in allen wässrigen Lösungen nachgewiesen und entfernt werden. Diese Lösungen können: Proteine, Plasmid-DNA, genomische DNA, RNA, Protein-Nukleinsäurekomplexe wie z.B. Phagen oder Viren, Saccharide, Impfstoffe, Arzneimittel, Dialysepuffer (Medizin), Salze oder andere durch Endotoxin-Bindung verunreinigte Substanzen enthalten.

Ein weiterer Aspekt der Erfindung sind Bakteriophagenproteine, an die sogenannte Tags, z.B. der Strep- oder der His-Tag, vorzugsweise an den N- oder C-Terminus des Proteins, besonders bevorzugt an den. C-Terminus, gekoppelt sind. Bevorzugt ist die Kopplung oder Verknüpfung der Tags mit den Bakteriophagenproteinen über DNA-Rekombinationstechnologie. Herstellung der Nukleinsäure, umfassend die Sequenz des Bakteriophagenproteins und des Tags und die Herstellung des Expressionsprodukts sind Stand der Technik und brauchen hier nicht gesondert erläutert zu werden. Ein weiterer Aspekt der Erfindung ist die Nukleinsäuresequenz, die ein Bakteriophagenprotein zusammen mit dem Strep- oder His-Tag codiert. Ein besonders bevorzugtes mit dem Strep- oder His-Tag modifiziertes Bakteriophagenprotein ist das p12-Protein vom Phagen T4, jedoch sind alle anderen Bakteriophagenproteine die an der Erkennnung und Bindung von Bakterien beteiligt oder dafür verantwortlich sind ebenfalls bevorzugt.

Ein weiterer Aspekt der Erfindung sind Bakteriophagenproteine, mit einem Tag, der ein oberflächenexponiertes Cystein zur spezifischen, gerichteten Biotinylierung aufweist, z.B. die Tags gemäß SEQ ID NO: 5, 6 und 7. Ein Beispiel für ein p12 mit Tag ist die in SEQ ID NO:8 aufgeführte Aminosäuresequenz. Bevorzugt ist ein p12 mit einem Tag, insbesondere mit einem Tag mit einem oberflächenexponierten Cystein, insbesondere ein p12 mit dem Tag gemäß SEQ ID NO: 6 und 7. Diese gerichtete Biotinylierung kann zusätzlich durch einen geeigneten Spacer oder Linker vermittelt werden. Ferner betrifft die vorliegende Erfindung die Aminosäuren mit einer Sequenz gemäß SEQ ID NO:5, 6 und 7. Ferner betrifft die vorliegende Erfindung die Nukleinsäuren, codierend die Aminosäuresequenz gemäß SEQ ID NO:5, 6 und 7.

Die erfindungsgemäßen Verfahren bieten gegenüber den Nachweis- und Reinigungsverfahren für und von Endotoxin Vorteile in der Performance entsprechender Anwendungen. Ferner ist die Herstellung von Antikörper gegen LPS-Herzoligosaccharide sehr schwierig, was entsprechende Verfahren auf Antikörper-Basis sehr teuer werden lässt.

Die folgenden Beispiele erläutern die Erfindung und sind nicht als einschränkend aufzufassen. Sofern nicht anders angegeben, wurden molekularbiologische Standardmethoden verwendet, wie z.B. von Sambrook et al., 1989, Molecular cloning: A Laboratory Manual 2. Auflage, Cold Spring Harbor Laboratory Press, Cold Spring Harbor, New York, beschrieben.

### 1. Glasgefäße, Plastikgefäße und Puffer

Für die Endotoxinentfernung wurden alle Glasgefäße durch Ausbacken bei 200°C (4h) entpyrogenisiert und ausschließlich pyrogenfreie Plastikmaterialen (z.B. Pipettenspitzen, Microtiterplatten) verwendet. Andere, nicht hitzebeständige Geräte oder Gefäße, wurden entweder mit 3% Wasserstoffperoxid behandelt oder mit 1% Natriumdeoxoycholat gewaschen. Anschließend wurde sie mit endotoxinfreiem Wasser gespült. Die Puffer wurden aus weitgehend endotoxinfreien Puffersubstanzen (Sigma) hergestellt und mit endotoxinfreiem Wasser angesetzt. Salze, wie z.B. NaCl, die auf 200°C erhitzt werden können, wurden ausgebacken (200°C, 4h). Für chromatographische Reinigungen verwendete Puffer wurden entgast und filtriert.

### 2. Endotoxinnachweis mittels LAL-Test

Endotoxin-Kontrollnachweise wurden mit einem chromogenen LAL-Test (Limulus-Amebocyte-Lysate Test, Charles-River Endosafe, Charleston, USA) entsprechend den Angaben des Herstellers durchgeführt. Zur Konzentrationsbestimmung wurden Endotoxin-Standards (Charles-River Endosafe, Charleston, USA) im Bereich von 0.005-50, bzw. 0.02-50 EU/ml eingesetzt. Die Absorptionsmessung bei 405 nm erfolgte in einem temperierbaren Mikrotiterplatten-Reader (Genios, Tecan GmbH).

### 3. Western-Blot zum p12-Nachweis

Der Nachweis von p12 im Überstand von mit Beads behandelten Proben bzw. in den Fraktionen der Affinitätschromatographie erfolgte durch Western Blots. Zum Teil wurden die Proteine vorher durch NaDOC/TCA-Fällung (Natriumdeoxycholat/Tetrachloracetat) aufkonzentriert. Die Proben wurden dazu auf 12%-igen SDS Gelen elektrophoretisch aufgetrennt und auf PVDF Membranen (Immobilon, Millipore) übertragen. Die Membranen wurden mit PBS 30 min gewaschen, mit 5% Milchpulver blockiert (1 h) und anschließend mit polyklonalem anti-p12 Antikörper inkubiert (1h, Verdünnung: 1: 1000). Nach Inkubation mit einem, mit alkalischer Phosphatase konjugierter Sekundärantikörper (Ziege-anti-Kaninchen IgG) erfolgte die Entwicklung der Proben mit BCIP/NBT (5-Brom-4-chloroindolylphosphat/Nitroblau-Tetrazoliumsalz).

### 4. Endotoxin-Reinigung

Die Reinigung von Endotoxin wurde nach der Vorschrift von Galanos, C., Lüderitz, O. & Westphal, O. 1969, Europ. J. Biochem. 9, 245-249 durchgeführt.

### Beispiel 5: Spezifische Kopplung von p12 an immobilisierte Jodoacetylreste:

Um eine gerichtete Bindung von p12 an die Oberfläche zu erreichen wurde die Aminosäure Serin an Position 3 des Strep-Tags gemäß SEQ ID NO:5 durch Cystein wie in Beispiel 12 ersetzt und das Protein über Jodoacetylreste, die bevorzugt freie Sulfhydrylreste binden, immobilisiert. Das resultierende p12 wurde p12S3C genannt.

Es wurde ein 1 ml Sulfolink Coupling Gel (Pierce) gegossen, mit 6 ml 1% Natriumdeoxycholat gewaschen und mit 6 ml Kopplungspuffer (50 mM Tris, 150 mM NaCl, 5 mM EDTA, pH 8.5) equilibriert. Anschließend wurden 1 ml p12S3C (=N-StrepS3Cp12) (1-1.5 mg/ml in Kopplungspuffer) injiziert, die Säule 15 min leicht geschüttelt, weitere 30 min ohne Schütteln bei Raumtemperatur inkubiert, und nochmals 1 ml p12S3C injiziert und die Inkubationsschritte wiederholt. Diese Kopplung von p12S3C wurde insgesamt 4 mal wiederholt, und anschließend die Säule mit 6 ml Kopplungspuffer gewaschen. Die Durchläufe wurden gesammelt und die jeweilige p12S3C Konzentration durch Absorptionsmessung bei 280 nm bestimmt. Es wurden 2.2-2.8 mg p12S3C pro ml Gel gebunden. Anschließend wurden überzählige Jodoacetylreste durch Inkubation (45 min) mit 1 ml Cystein (50 mM in 50 mM Tris, 5 mM EDTA, pH 8.5) blockiert. Nach Waschen der Säule mit 16 ml IM NaCl und 16 ml 20 mM Hepes, 150 mM NaCl pH 7.5 war die Säule fertig zum Gebrauch.

Die Fähigkeit dieses Gels Endotoxin aus Proteinlösungen zu entfernen, wurde mit BSA (2-4 mg/ml), Carbon Anhydrase (1-2 mg/ml) und Lysozym (3-4 mg/ml) getestet. BSA und Lysozym Lösungen wurden mit Endotoxin von E. coli 055:B5 (Charles-River Endosafe, Charleston, USA) oder E. coli HMS 174 gespickt (100-1000 EU/ml), während die Carbon Anhydrase nicht mit zusätzlichem Endotoxin versetzt wurde. Es wurden jeweils 0.5 ml Proteinlösung auf die Säule gegeben, 1 Stunde bei Raumtemperatur inkubiert und anschließend die Säule mit Puffer gewaschen. Die Proteine wurden fraktionsweise gesammelt und der Endotoxingehalt vor und nach der Säule mittels eines chromogenen LAL-Tests (Charles-River Endosafe, Charleston, USA) bestimmt. Außerdem wurde die Proteinwiederfindung durch Absorptionsmessungen bei 280 nm ermittelt. Die Endotoxine konnten aus allen 3 Proteinlösungen fast vollständig (93-99%) entfernt werden, wie in Fig. 2A gezeigt. Außerdem konnten die Proteine weitgehend von der Säule eluiert werden (80-99%, Fig. 2B). Die Säule wurde abschließend mit 5 mM EDTA, 20 mM Hepes, 150 mM NaCl, pH 7.5 regeneriert. Um Verunreinigungen der Proteinfraktionen nach dem Lauf über die Säule durch sich ablösendes p12 auszuschließen, wurden die Fraktionen mittels der Western Blot Technik auf p12 untersucht. Es konnte kein p12 in den Fraktionen nachgewiesen werden.

### Beispiel 6: Unspezifische Kopplung von p12 an NHS-aktiviertes Trägermaterial:

N-hydroxysuccinimid (NHS) wird aus Verbindungen durch primäre Aminoreste verdrängt und deshalb zum Koppeln von Proteinen an Oberflächen benutzt. NHS-aktivierte Sepharose Säulen (HiTrap NHS-activated HP, 1 ml, Amersham-Pharmacia-Biotech) wurden zunächst mit 6 ml eiskalter 1 mM Salzsäure gewaschen. Anschließend wurden bei Raumtemperatur 10-15 ml p12S3C (1.0-3.5 mg/ml) in 0.2 M NaHCO₃, 0.5 M NaCl, pH 8.3 zirkulär über die Säule gepumpt (Flussrate 0.8 ml/min). Nach 60 min wurde der Durchlauf fraktionsweise gesammelt und die Säule mit 6 ml Puffer gewaschen. Aus diesen Fraktionen wurde das NHS durch Entsalzen der Lösung über HiTrap-Desalting Säulchen (5 ml, Amershanl-Pharmacia-Biotech) abgetrennt und anschließend die p12-Menge durch Absorptionsmessung bei 280 nm bestimmt. 20-25 mg p12S3C wurden an die Säule gebunden. Die Säule wurde nach der Kopplung entsprechend den Herstellerangaben wiederholt mit jeweils 6 ml Blockierungspuffer (0.5 M Ethanolamin, 0.5 M NaCl, pH 8.3) und Waschpuffer (0.1 M Acetat, 0.5 M NaCl, pH 4.0) gespült. Anschließend wurde die Säule mit 6 ml Gebrauchspuffer (20 mM Hepes, 150 mM NaCl, pH 7.5 oder 20 mM Tris, 150 mM NaCl, pH 8.5) equilibriert.

Die Endotoxinentfernung über diese Säule wurde mit Lysozymlösungen (3-4 mg/ml in 20 mM Hepes, 150 mM NaCl, pH 7.5 oder 20 mM Tris, 150 mM NaCl, pH 8.5) getestet. Die Lysozymlösungen wurden mit Endotoxin von E. coli HMS 174 gespickt (-500 EU/ml). Es wurden 0.5 ml Proteinlösung auf die Säule gegeben, 1 Stunde bei Raumtemperatur inkubiert und anschließend die Säule mit Puffer gewaschen. Das Lysozym wurden fraktionsweise gesammelt und der Endotoxingehalt vor und nach der Säule mittels eines chromogenen LAL-Tests (Charles-River Endosafe, Charleston, USA) bestimmt. Außerdem wurde die Proteinwiederfindung durch Absorptionsmessungen bei 280 nm ermittelt. Die Endotoxine wurden zu 85-90% aus der Lösung entfernt, wie in Fig. 3A gezeigt und 85-90% des Lysozyms konnten durch Waschen mit Gebrauchspuffer wieder von der Säule eluiert werden (Fig. 3B). Die Säule wurde anschließend mit 6 ml 5 mM EDTA, 20 mM Hepes, 150 mM NaCl, pH 7.5 und 6 ml 1 M NaCl gewaschen. Um Verunreinigungen der Proteinfraktionen nach dem Lauf über die Säule durch sich ablösendes p12 auszuschließen, wurden die Fraktionen mittels der Western Blot Technik auf p12 untersucht. Es konnte kein p12 in den Fraktionen nachgewiesen werden.

### Beispiel 7: Gerichtete Kopplung von p12 an über Diaminoethan und N-Succinimidyl-iodoacetat (SIA) als Spacer an NHS-aktiviertes Trägermaterial-Säule.

Um eine gerichtete Bindung an das Chromatographie Trägermaterial zu erreichen wurde ein bifunktioneller Linker an NHS-aktivierte Oberfläche gebunden, der eine Kopplung von p12S3C über dessen freies Cystein und Jodoacetylreste des bifunktionalen Linkers ermöglicht.

NHS-aktivierte Sepharose Säulen (HiTrap NHS-activated HP, 1 ml, Amersham-Pharmacia-Biotech) wurden zunächst mit 6 ml eiskalter 1 mM Salzsäure gewaschen, danach 1 ml Ethylendiamin (10 mg/ml in 0.2 M NaHCO₃, 0.5 M NaCl, pH 8.3) injiziert und die Säule 30 min bei Raumtemperatur inkubiert. Nach Blockieren überzähliger NHS-Gruppen mit Ethanolamin (0.5 M Ethanolamin, 0.5 M NaCl, pH 8.3) und Waschen (0.1 M Acetat, 0.5 M NaCl, pH 4.0) der Säule wurde die Säule mit 6 ml Boratpuffer ( 50 mM Natriumborat, 150 mM NaCl, 5 mM EDTA, pH 8.3) equilibriert. Anschließend wurde 30 min lang10 ml N-Succinimidyl-iodoacetat (SIA, Pierce, 200 µl SIA-Stammlösung in 10 ml Boratpuffer; SIA-Stammlösung: 1.4 mg SIA in 1 ml DMSO) zirkulär über die Säule gespült. Die Säule wurde danach mit 6 ml Boratpuffer gewaschen und 1 Stunde lang p12S3C (1 mg/ml, 50 ml in Boratpuffer) über die Säule gespült. Überschüssige Iodoacetylreste wurden mit 1 ml Cysteinlösung (5mM Cystein in Boratpuffer, 15 min bei Raumtemperatur inkubieren) abgesättigt, bevor die Säule mit den Gebrauchspuffern (20' mM Hepes, 150 mM NaCl, pH 7.5 oder 50 mM Tris, 150 mM NaCl, pH 8.5) equilibriert wurden. Die Kopplungsreaktionen mit SIA wurden im Dunkeln durchgeführt.

Die Endotoxinentfernung über diese Säule wurde mit Lysozymlösungen (3-4 mg/ml in 20 mM Hepes, 150 mM NaCl, pH 7.5 oder 20 mM Tris, 150 mM NaCl, pH 8.5) getestet. Die Lysozymlösungen wurden mit Endotoxin von E. coli HMS 174 gespickt (~500 EU/ml). Es wurde 0.5 ml Proteinlösung auf die Säule gegeben, 1 Stunde bei Raumtemperatur inkubiert und anschließend die Säule mit Puffer gewaschen. Das Lysozym wurde fraktionsweiße gesammelt und der Endotoxingehalt vor und nach der Säule mittels eines chromogenen LAL-Tests (Charles-River Endosafe, Charleston, USA) bestimmt. Außerdem wurde die Proteinwiederfindung durch Absorptionsmessungen bei 280 nm ermittelt. Die Endotoxine wurden zu 90% aus der Lösung entfernt, wie in Fig. 3A gezeigt und 75-85% des Lysozyms konnten durch Waschen mit Gebrauchspuffer wieder von der Säule eluiert werden (Fig. 3B). Die Säule wurde anschließend mit 6 ml 5 mM EDTA, 20 mM Hepes, 150 mM NaCl, pH 7.5 und 6 ml 1 M NaCl gewaschen. Um Verunreinigungen der Proteinfraktionen nach dem Lauf über die Säule durch sich ablösendes p12 auszuschließen, wurden die Fraktionen mittels der Western Blot Technik auf p12 untersucht. Es konnte kein p 12 in den Fraktionen nachgewiesen werden.

### Beispiel 8: Entfernung von Endotoxin aus einer BSA-Lösung im Durchflussverfahren

Hi-Trap-NHS aktivierte Sepharose (Amersham Biosciences, Uppsala, Schweden) wurde nach Vorschrift des Herstellers unspezifisch über primäre Aminogruppen mit p12 gekoppelt. Dabei wurden 8 mg p12/ml Gelmaterial kovalent immobilisiert. Die so erhaltene 1 m1 Chromatographiesäule wurde mit einer Flussrate von 1 ml/min mit 10 ml Puffer A (20 mM HEPES pH 7.5, 150 mM NaCl, 0.1 mM CaCl2) äquilibriert. Im Anschluß wurden 4 ml einer BSA Lösung (11.5mg BSA (Carl Roth GmbH, Deutschland) / ml Puffer A) aufgetragen (Injektion: I) und der Durchlauf (E) in 2,5 ml Fraktionen gesammelt. Die Säule wurde anschließend mit 15 ml Puffer A gewaschen und das an die Säule gebundene Endotoxin wurde mit 7 ml Puffer B (20 mM HEPES pH 7.5, 150 mM NaCl, 2 mM EDTA) eluiert. Bei Waschen und Elution wurden jeweils 2 ml Fraktionen gesammelt. Nach jedem Experiment wurde die Säule mit 20 ml Puffer C (20 mM HEPES pH 7.5, 150 mM NaCl, 2 mM EDTA, 0.1 % Natriumdesoxycholat) regeneriert. Die Endotoxin-Konzentration wurde durch einen chromogenen Limulus Amebocyte Lysate (LAL) Test (Charles-River Endosafe, Charleston, USA), nach Vorschrift des Herstellers bestimmt. Die Bestimmung der Proteinkonzentration erfolgte durch Messung der UV-Absorption. Die Endotoxin-Entfemungseffizienz betrug zwischen 95-99% und der Proteinverlust betrug etwa 6-10 %.

### Beispiel 9: Entfernung geringer Endotoxinmengen aus Puffer mittels unspezifisch gekoppeltem p12.

20 ml NHS-aktivierte Sepharose 4 FastFlow (Amersham Biosciences) wurden zunächst mit eiskalter Salzsäure gewaschen und anschließend mit 292 mg p12 (7 mg/ml in 25 mM Citrat pH 7.0) 4 Stunden unter schütteln bei Raumtemperatur inkubiert. Anschließend wurde die Sepharose mit 7 x 80 ml 5 mM Citrat pH 2.0 gewaschen und jeweils 1 ml der Waschfraktionen gegen 5 mM Citrat pH 2.0 dialysiert. Diese Dialysate wurden benutzt, um das überschüssige p12 in den Waschfraktionen mittels Absorptionsmessung bei 280 nm zu quantifizieren. Es wurde eine Beladungsdichte von 8.7 mg p12 pro 1ml Sepharose bestimmt. Nicht abreagierte NHS-Reste wurden durch 12 h Inkubation der Sepharose mit 1M Tris pH 8.0 abgesättigt. Mit diesem Säulenmaterial wurden Säulen mit 2 ml Volumen gegossen und diese bei 4°C in 20% Ethanol bis zum Gebrauch gelagert.

In 3 Parallelversuchen wurde jeweils 4 ml Endotoxin Lösung (S) auf eine Säule aufgetragen (siehe Fig. 9). Die Endotoxin Lösung bestand aus Endotoxin von E. coli O55:B5 (Charles-River Endosafe, Charleston, USA) in Equilibrationspuffer (20 mM Hepes, 150 mM NaCl, 0.1 mM CaCl₂, pH 7.5). Die Endotoxin Konzentration dieser Lösung lag bei 4.6 EU/ml.

Die Säulen wurde zunächst mit 12 ml Regenerationspuffer (20 mM Hepes, 150 mM NaCl, 2 mM EDTA, pH 7.5) und anschließend mit 12 ml Equilibrationspuffer gespült. Anschließend wurde nochmals Equlilibrationspuffer auf die Säule gegeben und 1ml fraktioniert.

Die Endotoxin Lösung wurden auf die Säulen aufgetragen (I) und Fraktionen von 5 ml und 2 ml gesammelt. Anschließend wurde die Säule mit 4 ml Regenerationspuffer (B) regeneriert. In den Durchlauffraktionen konnte kein Endotoxin detektiert werden, d.h. die Endotoxin Verunreinigungen konnten in allen drei Experimenten vollständig entfernt werden.

### Beispiel 10: unspezifische Kopplung von biotinyliertem p12 an magnetische Streptavidin-Beads.

p12 (3 mg/ml in PBS, 0.05% Tween20) wurde mit Sulfo-NHS-LC-LC-Biotin (Pierce), im Verhältnis 1:10 bis 1:20 eine Stunde bei RT inkubiert und anschließend gegen Puffer (z.B. PBS oder 20 mM Hepes, 150 mM NaCl, 5 mM EDTA, pH 7.5) dialysiert. NHS-aktiviertes Biotin bindet dabei an primäre Aminoreste von p12. Anschließend wurden zu 1ml Streptavidin Beads (MagPrep Streptavidin Beads, Merck) 50 µl biotinyliertes p12 (1 mg/ml) gegeben, 2h bei Raumtemperatur geschüttelt und anschließend überschüssiges p 12 durch viermaliges Waschen mit 1.5 ml 20 mM Tris, 10 mM EDTA, pH 7.5 entfernt.

Die Endotoxinentfernung wurde mit Puffer (20 mM Hepes, 150 mM NaCl, pH 7.5) und Proteinlösungen (0.1 mg/ml BSA, 0.1 mg/ml Lysozym, 0.1 mg/ml Carbon Anhydrase in 20 mM Hepes, 150 mM NaCI, pH 7.5) getestet. Der Puffer sowie die BSA- und Lysozym-Lösung wurde mit 5 EU/ml (Endotoxin aus E. coli O55:B5, Charles-River Endosafe, Charleston, USA) gespickt. Die Carbon Anhydrase Lösung enthielt etwa 1 EU/ml. Zu 200 µl Puffer bzw. Proteinlösung wurden 25 µl magnetische Beads mit immobilisiertem p12 gegeben, durch auf- und abpipettieren vermischt und 30 min bei Raumtemperatur inkubiert. Die Beads wurden mit Hilfe eines Magneten aus der Lösung entfernt, der Überstand abpipettiert. Der Endotoxingehalt von unbehandelten Proben und mit Beads inkubierten Proben wurde anschließend mit dem LAL-Test bestimmt und die Proteinwiederfindung durch Absorptionsmessung bei 280 nm bestimmt. Aus Puffer ließ sich das Endotoxin praktisch vollständig entfernen (99.9 % Endotoxinentfemung, Fig. 4A) und auch aus den Proteinlösung wurde das Endotoxin um 70-92% (Fig. 4B) abgereichert. Die Proteinwiederfindung lag zwischen 57% und 99% (BSA: 87 %,Carbon Anhydrase: 99%, Lysozym: 57 %; Fig. 4B).

### Beispiel 11: unspezifische Kopplung von biotinyliertem p12 an immobilisiertes Streptavidin.

P12 (3 mg/ml in PBS, 0.05% Tween20) wurde mit Sulfo-NHS-LC-LC-Biotin (Pierce), im Verhältnis 1:10 bis 1:20 eine Stunde bei RT inkubiert und anschließend gegen Puffer (z.B. PBS oder 20 mM Hepes, 150 mM NaCl 5 mM EDTA, pH 7.5) dialysiert. NHS-aktiviertes Biotin bindet dabei an primäre Aminoreste von p12. Das biotinylierte p12 wird anschließend 1 h bei Raumtemperatur mit Streptavidin beladenen Chromatographiematerial (ImmunoPure immobilized Streptavidin: 6% quervernetzte Agarose Beads) inkubiert und überschüssiges p12 durch Waschen mit PBS entfernt.

Die Endotoxinentfernung wurde mit Puffer (20 mM Tris, 150 mM NaCl, pH 8.0) und BSA (0.5 mg/ml in 20 mM Tris, 150 mM NaCl, pH 8.0) getestet. Je 1 ml Puffer bzw. BSA-Lösung wurden mit 10 EU/ml gespickt, 50 µl p12-Agarose zugegeben, 1 Stunde bei Raumtemperatur geschüttelt. Die p12. Agarose wurde anschließend abzentrifugiert und die Endotoxin- und Proteinkonzentration im Überstand gemessen. Aus dem Puffer konnten 99% und aus der BSA-Lösung 86 % Endotoxin entfernt werden (Fig. 5). BSA konnte zu 90 % wiedergefunden werden.

### Beispiel 12: Untersuchungen über die p12-Endotoxin Bindung mittels Oberflächen-Plasmon-Resonanz-Messungen

Die Bindung von p12 an Endotoxin oder an Bakterien, über die Lipopolysaccharide in der äußeren Zellmembran, wurde mittels Oberflächen-Plasmon-Resonanz Messungen untersucht (Biacore J). Um die Dissoziationskonstante (K_{d}) zu ermittelt, wurde Endotoxin von E. coli O55:B5 (Sigma) auf einem hydrophoben HPA-Chip entsprechend der Anleitung des Herstellers immobilisiert und p12 in verschiedenen Konzentrationen injiziert (Fig. 6A). Die Bindung wird in relativen "Response Units" (RU) gemessen die Gleichgewichtswerte gegen die dazugehörigen p12-Konzentrationen aufgetragen (Fig. 6B). Durch anpassen der Langmuirschen Adsorptionsisotherme (RU = (RUₘₐₓ*[p12])/([p12]+K_{d})) an diese Daten wurde der K_{d}-Wert ermittelt (Tabelle 1). Für die Messungen wurden endotoxinfreie Puffer verwendet. Für pH-Werte zwischen 6 und 10 wurden K_{d}-Werte im Bereich von 10⁻⁷ bis 10⁻⁹ M ermittelt (Tabelle 1). Die Bindung wurde durch Injektion von 1mM oder 5 mM EDTA wiederaufgehoben und der Chip regeneriert.

**Tabelle 1: Dissoziationskonstanten von Endotoxin an p12 in Abhängigkeit von dem pH-Wert der Lösung.**

| **pH** | **Kd** |
|---|---|
| 6,00 | 3,09E-07 |
| 7,50 | 6,85E-08 |
| 8,00 | 5,86E-08 |
| 8,50 | 7, 86 E-08 |
| 9,00 | 3,29E-08 |
| 10,00 | 1,55E-07 |

Um die Bindung von Bakterien an p12 zu untersuchen, wurde biotinyliertes p12 auf Streptavidin-Chips immobilisiert und verschiedene E. coli Stämme injiziert. Die Bakterien wurden für die Messungen in PBS aufgenommen. Es wurden E. coli Stämme verwendet, die Lipopolysaccharide mit unterschiedlichen Polysaccharid-Anteilen besitzen. Der Polysaccharidteil besteht aus einer "Herz"-Region, die mit dem Lipid A verknüpft ist und dem sogenannten O-Antigen. Das O-Antigen variert sehr stark zwischen verschiedenen Bakterienarten und auch Bakterienstämmen, während die "Herz"-Region stark konserviert ist. Stämme, die die "Herz"-Region und O-Antigen (z.B. E. coli), sowie Stämme die eine vollständige "Herz"-Region (E. coli D21) besitzen wurden von p12 gebunden, während Stämme mit einem stark verkürzter "Herz"-Region (z.B. E. coli D21f2) nicht mehr von p12 erkannt wurden (Fig. 6C). Die Bindung konnte durch EDTA (5 mM) wieder gelöst und der Chip regeneriert werden.

### Beispiel 13: rekombinante p12-Konstrukte

1. Konstruktion von p12 mit N-terminalem Strep-Tag (N-Strep-p12): Mittels PCR wurde an das 5'-Ende des T4p12-Gens die Nukleotidsequenz für den Strep-Tag (US patent 5,506,121) eingeführt. Hierfür wurde für das 5'-Ende des p12-Gens ein Primer konstruiert (5'-GAA GGA ACT AGT CAT ATG *GCT AGC TGG AGC CAC CCG CAG TTC GAA AAA GGC GCC* AGT AAT AAT ACA TAT CAA CAC GTT-3' (SEQ ID NO:1), der die Nukleotidsequenz des Strep-Tags an seinem 5'-Ende beinhaltet (kursiv in der Sequenz) und eine Restriktionsschnittstelle (*Nde*I*,* unterstrichen in der Sequenz) derart besitzt, dass das Gen im richtigen Leseraster in das Expressionsplasmid eingesetzt werden kann. Für das 3'-Ende des p12-Gens wurde ein Primer konstruiert, der hinter dem p12-Gen eine *Bam*H I Restriktionsschnittstelle (kursiv in der Sequenz) einführt (5'-ACG CGC AAA GCT TGT CGA CGG ATC CTA TCA TTC TTT TAC CTT AAT TAT GTA GTT-3'), (SEQ ID NO:2). Die PCR wurde mit 40 Cyclen (1 min 95°C, 1 min 45°C und 1 min 72°C) durchgeführt. Der PCR-Ansatz wurde mit den Restriktionsendonukleasen *Nde*I und *Bam*HI geschnitten und das gewünschte Fragment nach Größenfraktionierung über ein Agarosegel und Elution aus dem Gel in die *Nde*I und *Bam*HI site des Expressionsplasmids pET21a eingesetzt. Die Sequenz des N-Strep-p12-Gens wurde über DNA-Sequenzierung auf seine Richtigkeit hin überprüft Die weiteren Schritte zum Plasmid pNS-T4p12p57 wurden wie von Burda, M.R. & Miller, S. (Eur J Biochem. 1999 265 (2), 771-778) für T4p12p57 beschrieben durchgeführt. Das Plasmid pNS-T4p12p57 wurde dann in den Expressionsstamm BL21 (DE3) transformiert.
2. Einfügen eines N-terminalen Cysteinrests in N-Strep-p12 (N-Strep-S3C-p12 und N-Strep-S14C-p12): Die Einfügung eines N-terminalen Cysteinrestes wurde wie unter 1. beschrieben durchgerührt, wobei dafür zwei neue Primer für das 5'-Ende konstruiert wurden. Für das N-Strep-S3C-p12 wurde der Primer 5'-GAA GGA ACT AGT CAT ATG *GCT TGT TGG AGC CAC CCG CAG TTC GAA AAA GGC GCC* AGT AAT AAT ACA TAT CAA CAC GTT-3' (SEQ ID NO:3), für das N-Strep-S14C-p12 wurde der Primer 5'-GAA GGA ACT AGT CAT ATG *GCT AGC TGG AGC CAC CCG CAG TTC GAA AAA GGC GCC* TGT AAT AAT ACA TAT CAA CAC GTT-3' (SEQ ID NO:4)verwendet.
3. Reinigung von N-Strep-p12 Protein: Der *E. coli* Stamm BL21(DE3) mit dem Plasmid pNS-T4p12p57 wurde in 2 1 Schüttelkulturen (LB-Medium mit Ampicillin 100 µg/ml) bis zu einer OD600 von 0.5-0.7 bei 37°C gezogen und die Expression des N-Strep-p12-Proteins wurde durch Zugabe von 1mM IPTG (Isopropyl-β-thio-galactopyranoside) induziert. Nach Inkubation bei 37°C für 4h wurden die Zellen abgeerntet. Geerntete Zellen aus 10 1 Kultur wurden in 50 ml Natriumphosphat, 20 mM pH 7.2, 2 mM MgSO4, 0.1 M NaCl aufgenommen, durch dreimalige French-Press-Behandlung (20.000 psi) aufgebrochen und anschließend 30 min bei 15.000 rpm (SS34) abzentrifugiert. Nach zweimaligem Waschen im gleichen Puffer wurde das N-Strep-p12 Protein aus dem Pellet das Pellet 3x mit durch Rühren für 30 min in 40 mM TrisHCl pH 8.0, 10 mM EDTA extrahiert, der Ansatz für 30 min bei 15.000 rpm (SS34) zentrifugiert und das abgelöste NS-p12 im Überstand bei 4°C gelagert. Die Extraktion wurde zweimal wiederholt, und die vereinigten Überstände wurden auf eine Streptactin-Affinitätssäule (15 ml), äquilibriert mit Puffer "W" (100 mM TrisHCl pH 8, 1 mM EDTA, 150 mM NaCl), aufgetragen (IBA GmbH, Göttingen). Nach Waschen mit 5 Säulenvolumina Puffer "W" wurde mit 3 Volumina Puffer "W" mit 2.5 mM Desthiobiotin in Puffer "W" eluiert. Nach mehrmaliger Dialyse gegen Puffer "W" und Aufkonzentration wurde über SDS-PAGE und UV-Spektroskopie (Burda et.al. 1999) die Konzentration und Reinheit von N-Strep-T4p12 ermittelt. Aus 10 Liter Kultur wurden so ca. 100 mg N-Strep-T4p12 gereinigt.

| Name | Sequenz des Tag | |
|---|---|---|
| Nstrep-p12 | MASWSHPQFEKGAS | SEQ ID NO: 5 |
| Nstrep-p12-S3C | MACWSHPQFEKGAS | SEQ ID NO: 6 |
| Nstrep-p12-S14C | MASWSHPQFEKGAC | SEQ ID NO: 7 |

## Patentansprüche

1. Verfahren zum Nachweis von Endotoxin, umfassend die Schritte:
a) Inkubieren einer Probe mit einem in Bakteriophagen vorkommenden Protein, das Bestandteile von Zellmembranen binden kann,
b) Nachweis von an Bakteriophagenproteine gebundenes Endotoxin.

2. Verfahren nach Anspruch 1, gegebenenfalls ferner umfassend nach Schritt a) und vor Schritt b) den zusätzlichen Schritt
a') Abtrennung der Bakteriophagenprotein-Endotoxin-Komplexe von der Probe.

3. Verfahren nach einem der Ansprüche 1 bis 2, wobei der Nachweis mittels spektroskopischer Verfahren durchgeführt wird.

4. Verfahren zur Entfernung von Endotoxin aus einer Probe, umfassend die Schritte:
a) Inkubation oder in Kontakt bringen einer Probe mit in Bakteriophagen vorkommenden Proteinen, die Bestandteile von Zellmembranen binden können und die unspezifisch oder gerichtet, an einem festen Träger immobilisiert sind, wobei die Ca²⁺-Konzentration in der Inkubation 0,1 µM bis 10 mM und/oder die Mg²⁺-Konzentration 0,1 µM bis 10 mM beträgt,
b) Trennen des Bakteriophagenprotein-Endotoxin-Komplexes von der Probe.

5. Verfahren nach Anspruch 4, wobei die Schritte a) und b) in einem Chromatographiesäulen-Durchflussverfahren durchgeführt werden.

6. Verfahren nach Anspruch 4, wobei der feste Träger Filtrationsmedien, Glaspartikel, Magnetpartikel, Zentrifugations-, Sedimentationsmaterialien oder Füllmaterialien für Chromatographiesäulen sind.

7. Verfahren nach Anspruch 4 bis 6, wobei die Bakteriophagenproteine über Kopplungsgruppen an dem festen Träger immobilisiert sind.

8. Verfahren nach Anspruch 7, wobei die Kopplungsgruppe ein Lektin, Rezeptor oder Anticalin ist.

9. Verfahren nach Anspruch 7, wobei die Kopplungsgruppe ein Streptavidin oder Avidin ist und die Bakteriophagenproteine mit Biotin oder einem Strep-Tag gekoppelt sind.

10. Verfahren nach Anspruch 4 bis 6, wobei die Bakteriophagenproteine kovalent über chemische Bindungen an dem festen Träger immobilisiert sind.

11. Verfahren nach einem der vorhergehenden Ansprüche, wobei das Bakteriophagenprotein einen Strep-Tag oder einen His-Tag aufweist.

12. Verfahren nach Anspruch 11, wobei der Tag eine Aminosäuresequenz gemäß SEQ ID NO. 5, 6 oder 7 aufweist.

13. Verfahren nach Anspruch 11 oder 12, wobei als Bakteriophagenprotein das p12-Protein des Phagen T4 verwendet wird.

14. Verfahren nach einem der Ansprüche 1 bis 3, wobei markiertes Endotoxin aus der Bindung mit einem Bakteriophagenprotein verdrängt wird und das markierte Endotoxin anschließend nachgewiesen wird.

## Claims

1. A method for detecting endotoxin, comprising the steps:
a) incubating a sample with a protein occurring in bacteriophages and being able to bind components of cell membranes,
b) detecting endotoxin bonded to bacteriophage proteins.

2. The method according to claim 1, further comprising after step a) and prior to step b) the additional step of
a') separating the bacteriophage protein-endotoxin complexes from the sample.

3. The method according to one of the claims 1 to 2, the detection being implemented by means of spectroscopic methods.

4. A method for removing endotoxin from a sample, comprising the steps:
a) incubating a sample with or bringing a sample in contact with proteins occurring in bacteriophages and being able to bind components of cell membranes, the proteins being immobilised on a permanent carrier, non specifically or directed, and wherein the Ca²⁺ concentration in the incubation being 0.1 µM to 10 mM and/or the Mg²⁺ concentration being 0.1 µM to 10 mM.
b) separating the bacteriophage protein-endotoxin complex from the sample.

5. The method according to claim 4, the steps a) and b) being implemented in a chromatography column throughflow method.

6. The method according to claim 4, the permanent carrier being filtration media, glass particles, magnetic particles, centrifugation materials, sedimentation materials or filling materials for chromatography columns.

7. The method according to claim 4 to 6, the bacteriophage proteins being immobilised on the permanent carrier via coupling groups.

8. The method according to claim 7, the coupling group being a lectin, receptor or anticalin.

9. The method according to claim 7, the coupling group being a streptavidin or avidin and the bacteriophage proteins being coupled with biotin or a Strep-tag.

10. The method according to claim 4 to 6, the bacteriophage proteins being immobilised on the permanent carrier covalently via chemical bonds.

11. The method according to anyone of the preceding claims, the bacteriophage protein having a Strep-tag or a His-tag.

12. The method according to claim 11, the tag having an amino acid sequence according to SEQ ID NO. 5, 6 or 7.

13. The method according claim 11 or 12, the p12 protein of the phage T4 being used as bacteriophage protein.

14. The method according to anyone of the claims 1 to 3, marked endotoxin being displaced from the bond with a bacteriophage protein and the marked endotoxin being subsequently detected.

## Revendications

1. Procédé pour la mise en évidence d'une endotoxine, comprenant les étapes suivantes :
a) incubation d'un échantillon avec une protéine provenant de bactériophage, qui peut se lier à des composants des membranes cellulaires,
b) mise en évidence d'une endotoxine liée à la protéine de bactériophage.

2. Procédé selon la revendication 1, comprenant le cas échéant également, après l'étape a) et avant l'étape b), l'étape supplémentaire suivante
a') séparation du complexe protéine de bactériophage-endotoxine de l'échantillon.

3. Procédé selon l'une quelconque des revendications 1 à 2, dans lequel la mise en évidence est effectuée par l'intermédiaire de procédés spectroscopiques.

4. Procédé pour la séparation d'une endotoxine d'un échantillon, comprenant les étapes suivantes :
a) incubation ou mise en contact d'un échantillon avec des protéines provenant de bactériophages, qui peuvent se lier aux composants des membranes cellulaires, et qui sont immobilisées de façon non spécifique ou dirigée sur un support solide, dans laquelle la concentration de Ca²⁺ au cours de l'incubation atteint 0,1 µM à 10 mM et/ou la concentration en Mg²⁺ atteint 0,1 µM à 10 mM,
b) séparation des complexes protéine de bactériophage-endotoxine de l'échantillon.

5. Procédé selon la revendication 4, dans lequel les étapes a) et b) sont effectuées par des colonnes de chromatographie à débit.

6. Procédé selon la revendication 4, dans lequel le support solide est constitué par des moyens de filtration, des particules de verre, des particules magnétiques, des matériaux de centrifugation, de sédimentation ou des matériaux de remplissage.

7. Procédé selon l'une quelconque des revendications 4 à 6, dans lequel les protéines de bactériophages sont immobilisées sur le support solide par des groupes de couplage.

8. Procédé selon la revendication 7, dans lequel le groupe de couplage est une lectine, un récepteur ou une anticaline.

9. Procédé selon la revendication 7, dans lequel le groupe de couplage est une streptavidine ou une avidine, et les protéines de bactériophages sont couplées à la biotine ou à un strep-tag.

10. Procédé selon l'une quelconque des revendications 4 à 6, dans lequel les protéines de bactériophages sont immobilisées par liaisons covalentes ou chimiques au support solide.

11. Procédé selon l'une quelconque des revendications précédentes, dans lequel la protéine de bactériophage présente un strep-tag ou un his-tag.

12. Procédé selon la revendication 11, dans lequel le tag présente une séquence d'acides aminés selon SEQ ID NO. 5, 6 ou 7.

13. Procédé selon la revendication 11 ou 12, dans lequel la protéine p12 du phage T4 est utilisée comme protéine de bactériophage.

14. Procédé selon l'une quelconque des revendications 1 à 3, dans lequel l'endotoxine marquée est déplacée de la liaison avec une protéine de bactériophage et. l'endotoxine marquée est ensuite mise en évidence.
